# EUROPEAN PATENT APPLICATION

(11) **EP 4 014 963 A1**
(43) Date of publication of application: **22.06.2022**
(21) Application number: 20306585.9
(22) Date of filing: 16.12.2020
(51) Int. Cl.: A61K 9/00, A61K 9/08, A61K 47/18, A61K 47/34, A61K 31/439, A61P 37/06, C08G 63/664, C08L 67/04

(54) **PHARMACEUTICAL COMPOSITION**

(71) Applicant: Medincell, 34830 Jacou (FR)
(72) Inventor: The designation of the inventor has not yet been filed
(74) Representative: D Young & Co LLP

(57) **Abstract**

The present invention provides a pharmaceutical composition comprising
(a) a triblock copolymer having the formula:

PLAv-PEGw-PLAx

wherein v and x are the number of repeat units ranging from 1 to 3,000 and w is the number of repeat units ranging from 3 to 300 and v=x or v≠x in an amount of 22 to 34 w/w % of the total composition;
(b) a diblock copolymer having the formula:

mPEGy-PLAz

wherein y and z are the number of repeat units with y ranging from 2 to 250 and z ranging from 1 to 3,000 in an amount of 5 to 9 w/w% of the total composition;
(c) tacrolimus or a pharmaceutically acceptable salt thereof in an amount of 8 to 32 w/w % of the total composition; and
(d) organic solvent in an amount of 30 to 62 w/w % of the total composition.

## Description

### FIELD OF THE INVENTION

The present invention relates to extended release pharmaceutical compositions comprising tacrolimus, and the use of said compositions for the therapeutic suppression of the immune system of a subject, in particular for the prophylaxis and/or treatment of an autoimmune disease or allograft rejection, in particular the prophylaxis and/or treatment of kidney transplant rejection.

### BACKGROUND OF THE INVENTION

Tacrolimus (also named TK506 or fujimycone) is a highly potent immunosuppressive agent with proven activity and efficacy in *in vitro* as well as *in vivo* studies. Tacrolimus has the following chemical structure: 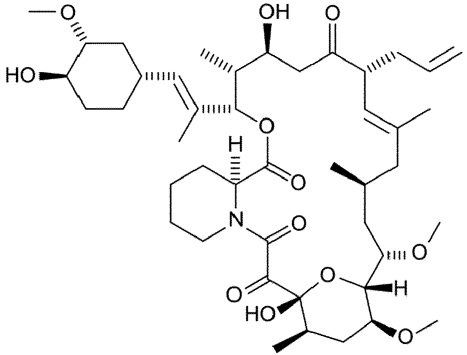 *In vitro,* tacrolimus inhibits the proliferative responses of lymphocytes to allogen and mitogen stimulation, the cytotoxic T-cell generation, the expression of the IL-2 receptor and the production of IL-2, IL-3 and IFN- γ (Kino et al. FK506, a Novel Immunosuppressant Isolated from a Streptomyces. II. Immunosuppressive Effect of FK-506 in vitro. J Antibiot 1987 and Scott et al. Tacrolimus: A Further Update of its Use in the Management of Organ Transplantation. Drugs 2003). The immunosuppressive efficacy of tacrolimus has been demonstrated in various animal models (dogs, rats, baboon and cynomolgus monkeys) of transplantation (Collier DS et al. Kidney Transplantation in the Dog Receiving FK-506. Transplant Proc. 1987; Murase N et al. Heterotopic Heart Transplantation in the Rat Receiving FK-506 Alone or with Cyclosporine. Transplant Proc 1987; Ochiai et al. Studies of the Effects of FK506 on Renal Allografting in the Beagle Dog. Transplantation 1987; Inamura et al. Prolongation of Skin Allograft Survival in Rats by a Novel Immunosuppressive Agent, FK506. Transplantation 1988; Todo et al. Orthotopic Liver Transplantation in Dogs Receiving FK-506. Transplant Proc 1987; Todo et al. Immunosuppression of Canine, Monkey, and Baboon Allografts by FK 506: with Special Reference to Synergism with Other Drugs and to Tolerance Induction. Surgery1988; Jiang et al. Tacrolimus Versus Cyclosporin A: a Comparative Study on Rat Renal Allograft Survival. Transpl Int. 1999 and Kinugasa et al. Efficacy of Oral Treatment with Tacrolimus in the Renal Transplant Model in Cynomolgus Monkeys. J Pharmacol Sci 2008).

Tacrolimus was first approved in the 1990's in the EU with Prograf^{®} oral capsule, then authorized as prolonged-release oral formulations by the EMA with Advagraf^{®} and Envarsus^{®}. Tacrolimus characteristics are therefore well documented and publicly available. Prograf^{®} 0.5 mg, 1 mg and 5 mg hard capsules (UK PL00166/0206 MAH Astellas Pharma Europe B.V.) are authorised for twice daily oral administration in the prophylaxis and treatment of transplant rejection in adult kidney, liver or heart allograft recipients.

Prolonged-release oral formulations for once daily administration (with a range of dosages) are authorised for the prophylaxis and treatment of transplant rejection in adult kidney, liver or heart allograft recipients: Advagraf^{®} prolonged-release hard capsules in 2007 (EMEA/H/C/000712), and Envarsus^{®} prolonged-release tablets in 2014 (EMEA/H/C/002655).

However, there is a need to provide an extended-released tacrolimus formulation in order to improve patient compliance and to offer greater convenience to the patient in comparison to the oral tacrolimus products currently on the market. Indeed, a retrospective cohort study showed that the patients who did not comply with their medication regimen were at increased risk for short-term allograft loss after a severe acute rejection (Al-Sheyyab et al. Association of Medication Non-Adherence with Short-Term Allograft Loss after the Treatment of Severe Acute Kidney Transplant Rejection. BMC Nephrol. 2019). Also, in a prospective study following 315 kidney allograft recipients, sixty kidneys progressed to failure in the follow-up period (median 31.4 months). Excluding four with missing information, 56 failures were attributed to four causes: rejection 36 (64%), glomerulonephritis 10 (18%), polyoma virus nephropathy 4 (7%) and intercurrent events 6 (11%). Every rejection loss had evidence of antibody-mediated rejection by the time of failure. Among rejection losses, 17 of 36 (47%) had been independently identified as patients who had not adhered to their medication by attending clinicians (Sellarés et al. Understanding the Causes of Kidney Transplant Failure: the Dominant Role of Antibody-Mediated Rejection and Nonadherence. Am J Transplant 2012).

There is also a need to provide a tacrolimus composition with fewer side effects and lower efficacy variability between patients.

Available tacrolimus is generally rapidly absorbed. Absorption is variable and the mean oral bioavailability of tacrolimus (investigated with the Prograf^{®} product) is in the range of 20% - 25% (individual range in adult patients 6% - 43%). The rate and extent of absorption of tacrolimus were reduced with food, both with the Prograf^{®} and Avagraf^{®} drug products. A strong correlation exists between AUC and whole blood trough levels at steady-state. Monitoring of whole blood trough levels therefore provides a good estimate of systemic exposure. Tacrolimus shows marked intra- and interpatient variability in absorption. Its absorption is not bile dependent but does depend on gastrointestinal transit time and is affected by the presence or absence of food, as well as the lipid content of food. In addition, age, gender, race, body mass index, time duration on tacrolimus, serum albumin, hematocrit, and presence of hepatitis B or C infection or other liver disease have all been shown to influence daily dosage requirements (Schiff et al. Therapeutic Monitoring of Calcineurin Inhibitors for the Nephrologist. Clin J Am Soc Nephrol 2007).

Data from literature has shown that when tacrolimus is administered intravenously or orally it is extensively metabolised by hydroxylation and demethylation in the liver and small intestine to at least nine metabolites, eight of which have been identified and characterized (Iwasaki. Review: Metabolism of Tacrolimus (FK506) and Recent Topics in Clinical Pharmacokinetics. Drug Metab Pharmacokinet 2007). Tacrolimus is predominantly metabolized by the CYP3A subfamily enzymes in the liver and small intestine, responsible for tacrolimus phase I metabolism. It is suggested that part of the between-patient variability in tacrolimus PK is due to the genetic variations in CYP3A5. A published article suggests that up to 30% of interindividual variability in apparent tacrolimus clearance is related to CYP3A5 polymorphisms (Rogier et al. Explaining Variability in Tacrolimus Pharmacokinetics to Optimize Early Exposure in Adult Kidney Transplant Recipients. Tjer Drug Monit 2009).

Recent studies have tried to address this through the use of controlled release formulations such as nano/micro particles or hydrogel (López-Farré et al. New Strategy of Tacrolimus Administration in Animal Model Based on Tacrolimus-Loaded Microspheres. Transp Immunol 2016; Myamoto et al. Pharmacokinetic and Immunosuppressive Effects of Tacrolimus-Loaded Biodegradable Microspheres. Liver transplantation 2004; Little et al. Micro and Nanoparticle Drug Delivery Systems for Preventing Allotransplant Rejection. Clin Immunol 2015). However, these articles relate to early stage research, and provide no clear evidence of an effective sustained release formulation of tacrolimus.

WO 2006/066063 and WO 2006/101972 describe injectable nanoparticulate formulations containing tacrolimus aimed to be used for the treatment of organ rejection and better controlling the pharmacokinetics parameters. However, although these applications describe the preparation of the formulations, no data in relation to the tacrolimus release profile, pharmacokinetics or proposed therapeutic uses is provided.

CN105919924 describes a tacrolimus sustained release temperature-sensitive gel for treating rheumatoid arthritis, organ grafting or autoimmunity eye diseases. The gel is prepared using a soluplus^{®} excipient. Sustained release of tacrolimus for a period of seven days was described.

However, there is a need for a composition that can provide release of tacrolimus at a therapeutically effective concentration for a period of at least 14 days, preferably at least 21 days, most preferably least 28 days.

### SUMMARY OF THE INVENTION

An aspect of the invention provides a pharmaceutical composition comprising
(a) a triblock copolymer having the formula:

   PLAᵥ-PEG_{w}-PLAₓ

   wherein v and x are the number of repeat units ranging from 1 to 3,000 and w is the number of repeat units ranging from 3 to 300 and v=x or v≠x in an amount of 22 to 34 w/w % of the total composition;
(b) a diblock copolymer having the formula:

   mPEG_{y}-PLA_{z}

   wherein y and z are the number of repeat units with y ranging from 2 to 250 and z ranging from 1 to 3,000 in an amount of 5 to 9 w/w% of the total composition;
(c) tacrolimus or a pharmaceutically acceptable salt thereof in an amount of 8 to 32 w/w % of the total composition; and
(d) organic solvent in an amount of 30 to 62 w/w % of the total composition.

A preferred embodiment provides a pharmaceutical composition comprising
(a) a triblock copolymer having the formula:

   PLAᵥ-PEG_{w}-PLAₓ

   wherein v and x are the number of repeat units ranging from 1 to 3,000 and w is the number of repeat units ranging from 3 to 300 and v=x or v≠x in an amount of 24 to 32 w/w % of the total composition;
(b) a diblock copolymer having the formula:

   mPEG_{y}-PLA_{z}

   wherein y and z are the number of repeat units with y ranging from 2 to 250 and z ranging from 1 to 3,000 in an amount of 6 to 8 w/w % of the total composition;
(c) tacrolimus or a pharmaceutically acceptable salt thereof in an amount of 10 to 30 w/w % of the total composition; and
(d) organic solvent in an amount of 30 to 60 w/w % of the total composition.

The compositions of the invention form an *"in situ* depot" which is a semi-solid, localized mass formed by precipitation of the pharmaceutical composition after injection of the composition into the subject. The pharmaceutical composition comprises copolymers which are substantially insoluble in aqueous solution. Thus, when the pharmaceutical composition contacts the aqueous environment of the human or animal body, a phase inversion occurs causing the composition to change from a liquid to a semi-solid, i.e. precipitation of the composition occurs, leading to formation of an *"in situ* depot".

The inventors have surprisingly found that the compositions of the invention enable the release of a therapeutically effective amount of tacrolimus for a period of at least 14 days, typically at least 21 days or preferably at least 28 days. This simplifies the dosage regimen for patients, allowing administration every 28 days rather than on a daily or twice daily basis as for marketed products, leading to improved patient compliance. When tacrolimus is used to prevent organ transplant rejection, improved patient compliance is understood to lead to a reduction in risk of organ transplant rejection. In addition, the composition of the invention is suitable for subcutaneous administration that is understood to lead to a reduction in observed side effects.

In a further aspect, the present invention provides a pharmaceutical composition for use in the therapeutic suppression of the immune system of a subject, wherein the use comprises administering the pharmaceutical composition to the subject, said composition comprising:
(a) a triblock copolymer having the formula:

   PLAᵥ-PEG_{w}-PLAₓ

   wherein v and x are the number of repeat units ranging from 1 to 3,000 and w is the number of repeat units ranging from 3 to 300 and v=x or v≠x in an amount of 22 to 34 w/w % of the total composition;
(b) a diblock copolymer having the formula:

   mPEG_{y}-PLA_{z}

   wherein y and z are the number of repeat units with y ranging from 2 to 250 and z ranging from 1 to 3,000 in an amount of 5 to 9 w/w % of the total composition;
(c) tacrolimus or a pharmaceutically acceptable salt thereof in an amount of 8 to 32 w/w % of the total composition; and
(d) organic solvent in an amount of 30 to 62 w/w % of the total composition.

In a preferred embodiment the invention provides a pharmaceutical composition for use in the therapeutic suppression of the immune system of a subject, wherein the use comprises administering the pharmaceutical composition to the subject, said composition comprising:
(a) a triblock copolymer having the formula:

   PLAᵥ-PEG_{w}-PLAₓ

   wherein v and x are the number of repeat units ranging from 1 to 3,000 and w is the number of repeat units ranging from 3 to 300 and v=x or v≠x in an amount of 24 to 32 w/w % of the total composition;
(b) a diblock copolymer having the formula:

   mPEG_{y}-PLA_{z}

   wherein y and z are the number of repeat units with y ranging from 2 to 250 and z ranging from 1 to 3,000 in an amount of 6 to 8 w/w % of the total composition;
(c) tacrolimus or a pharmaceutically acceptable salt thereof in an amount of 10 to 30 w/w % of the total composition; and
(d) organic solvent in an amount of 30 to 60 w/w % of the total composition.

In a preferred embodiment, for the triblock copolymer w is an integer from 20 to 25 and v and x are each an integer from 40 to 50, preferably wherein w is about 22 and v and x are each about 45; or wherein w is an integer from 20 to 25 and v and x are each an integer from 60 to 75, preferably wherein w is about 22 and v and x are each about 68.

Typically, for the triblock copolymer the molecular weight of the PEG repeat unit is 1 kDa, and the lactic acid/ethylene oxide molar ratio is 4 or 6.

In a preferred embodiment, for the diblock copolymer y is an integer from 7 to 10 and z is an integer from 60 to 85, preferably wherein y is about 8 and z is about 68; or wherein y is an integer from 43 to 47 and z is an integer from 129 to 141, preferably wherein y is about 45 and z is about 136.

Typically, for the diblock copolymer the molecular weight of the PEG repeat unit is 0.35 kDa and the lactic acid/ethylene oxide molar ratio is 8.5; or the molecular weight of the PEG repeat unit is 2kDa and the lactic acid/ethylene oxide molar ratio is 3.

In one embodiment of the composition or composition for use for the triblock copolymer the molecular weight of the PEG repeat unit is 1 kDa and the lactic acid/ethylene oxide molar ratio is 4.

In another embodiment of the composition or composition for use for the triblock copolymer the molecular weight of the PEG repeat unit is 1 kDa and the lactic acid/ethylene oxide molar ratio is 6.

In one embodiment of the composition or composition for use for the diblock copolymer the molecular weight of the PEG repeat unit is 0.35 kDa and the lactic acid/ethylene oxide molar ratio is 8.5.

In another embodiment of the composition or composition for use for the diblock copolymer the molecular weight of the PEG repeat unit is 2 kDa and the lactic acid/ethylene oxide molar ratio is 3.

Typically, a plurality of doses of the composition are administered to the subject. The compositions are typically administered over the lifetime of the subject.

In preferred embodiments, the time period between administration of consecutive doses is at least 14 days, preferably 21 days, most preferably at least 28 days. Typically, the composition is administered to the subject on a 4 weekly or monthly basis.

Typically, the use comprises parenteral administration of the composition, preferably subcutaneous injection of the composition.

In a preferred embodiment the use comprises injecting the composition using a needle and syringe, optionally using an injection device.

Typically, the dose of tacrolimus administered to the subject is controlled by adjusting the volume of the composition administered to the subject.

Preferably the composition releases a therapeutic concentration of tacrolimus into the blood plasma of the subject for at least 14 days, preferably at least 21 days, most preferably at least 28 days after administration of the composition. Typically, the therapeutic concentration of tacrolimus in the blood plasma of the subject is 5 to 20 ng/mL, with the proviso that the concentration of tacrolimus can be higher than 20 ng/mL for a period of up to 48 hours.

In a preferred embodiment the use comprises maintaining the daily AUC in the range of from about 130 to 475 ng.h/mL.

Typically, the use comprises maintaining the daily AUC ratio below a maximum value in the range of from about 3.60 to about 3.70, preferably below about 3.65.

In a preferred embodiment the composition comprises a dose concentration of tacrolimus of from about 115 to about 345 mg/mL, preferably about 230 mg/mL.

Typically the dose of tacrolimus is administered in a volume of from about 0.1 mL to about 2 mL, optionally from about 0.1 mL to about 1.5 mL, optionally from about 0.1 mL to about 1 mL, optionally from about 0.1 mL to about 0.5 mL.

The composition may be injected over a period of about 1 second to about 2 minutes, optionally about 1 second to 1 minute, optionally about 1 second to about 30 seconds, optionally about 1 second to about 20 seconds, optionally about 1 second to about 10 seconds, optionally about 1 to about 5 seconds.

Most preferably, the use comprises prophylaxis and/or treatment of allograft rejection in a subject who has received an allograft. The allograft may be an organ, tissue or cells. Preferably, the allograft is a liver, kidney or heart, most preferably a kidney.

In an alternative embodiment, the use comprises prophylaxis and/or treatment of an autoimmune disease. The use may comprise the prophylaxis and/or treatment of Lupus Nephritis.

The composition of the invention is suitable for forming a depot when injected into the body.

Typically the organic solvent is dimethyl sulfoxide.

In a preferred embodiment of the compositions of the invention, the weight ratio of the triblock copolymer to the diblock copolymer is about 4:1.

In a preferred embodiment, the composition comprises about 10 to 30 w/w % tacrolimus, optionally about 10 to 20 w/w % tacrolimus, optionally about 20 w/w % tacrolimus.

In a preferred embodiment the composition or composition for use comprises about 24 to 32 w/w % of the triblock copolymer, optionally 26 to 30 w/w % of the triblock copolymer, optionally about 27 to 29 w/w % of the triblock copolymer, optionally about 28 w/w% of the triblock copolymer.

In a preferred embodiment the composition or composition for use comprises about 6 to 8 w/w% of the diblock copolymer, optionally about 7 to 8 w/w% of the diblock copolymer, optionally about 7 w/w% of the diblock copolymer.

Typically the composition or composition for use comprises about 30 to 60 w/w%, optionally about 40 to 50 w/w%, optionally about 45 to 50 w/w%, optionally about 45 w/w% dimethyl sulfoxide.

The composition or composition for use may have a dynamic viscosity of from about 200 mPa.s. to about 3000 mPa.s, optionally from about 1000 mPa.s to about 3000 mPa.s, optionally from about 1000 to about 2000 mPa.s, optionally from about 1500 to about 1700 mPa.s.

The composition or composition for use may further comprise one or more pharmaceutically-acceptable excipients.

In a particularly preferred embodiment the composition or composition for use comprises 27-29 w/w % of the triblock copolymer, 6-8 w/w % of the diblock copolymer, 44-46 w/w % dimethyl sulfoxide, and 19-21 w/w % tacrolimus, and wherein for the triblock copolymer the molecular weight of the PEG repeat unit is 1 kDa and the lactic acid/ethylene oxide molar ratio is 4, and for the diblock copolymer the molecular weight of the PEG repeat unit is 2 kDa and the lactic acid/ethylene oxide molar ratio is 3.

In another particularly preferred embodiment the composition or composition for use comprises 23-25 w/w % of the triblock copolymer, 5-7 w/w % of the diblock copolymer, 59-61 w/w % dimethyl sulfoxide, and 9-11 w/w % tacrolimus, and wherein for the triblock copolymer the molecular weight of the PEG repeat unit is 1 kDa and the lactic acid/ethylene oxide molar ratio is 4, and for the diblock copolymer the molecular weight of the PEG repeat unit is 2 kDa and the lactic acid/ethylene oxide molar ratio is 3.

In a further aspect, the invention provides a method of therapeutic suppression of the immune system of a subject as described above, the method comprising the step of administering a composition as defined above to the subject.

In a further aspect, the present invention provides a method of preventing or treating allograft rejection in a subject who has received an allograft, the method comprising the step of administering a composition as defined in any preceding claim to the subject.

### BRIEF DESCRIPTION OF THE DRAWINGS

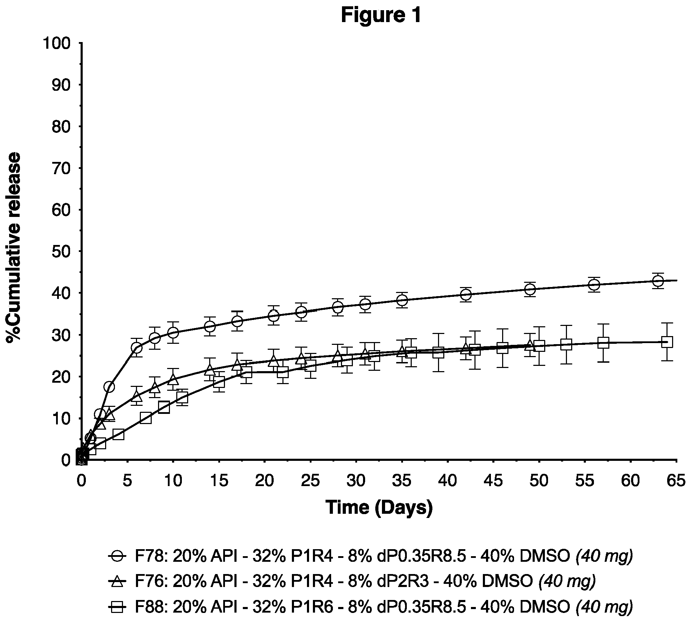

Figure 1 shows the *in vitro* cumulative release of tacrolimus over time from three different formulations (F76, F78 and F88). Formulations are loaded with different triblocks: P1R4 or P1R6 and different diblocks: dP0.35R8.5 or dP2R3 (TB/DB = 4/1). The copolymer content and the API loading are fixed at 40 and 20% (w/w %) respectively within all the formulations.

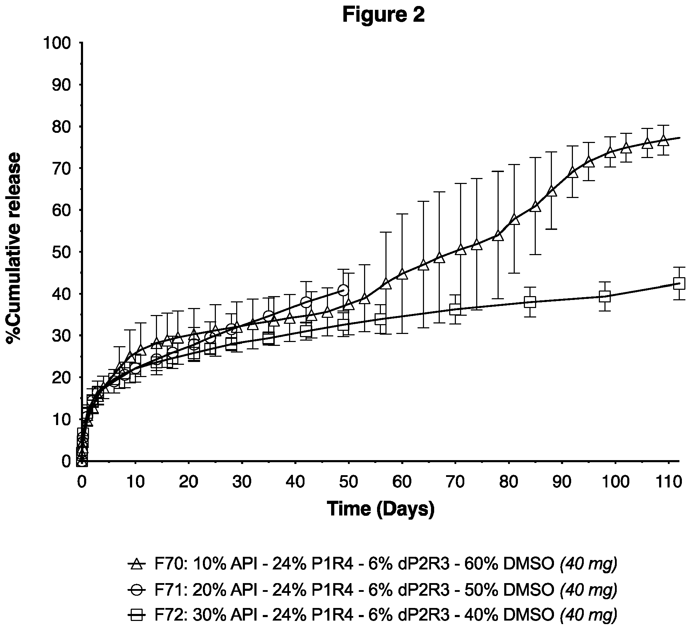

Figure 2 shows the *in vitro* cumulative release of tacrolimus over time from three different formulations (F70, F71 and F72). Formulations are loaded with P1R4 and dP2R3 (TB/DB = 4/1). The copolymer content is identical and fixed at 30% (w/w %) for all the formulations, but the API loading is different: 10, 20 or 30% (w/w %).

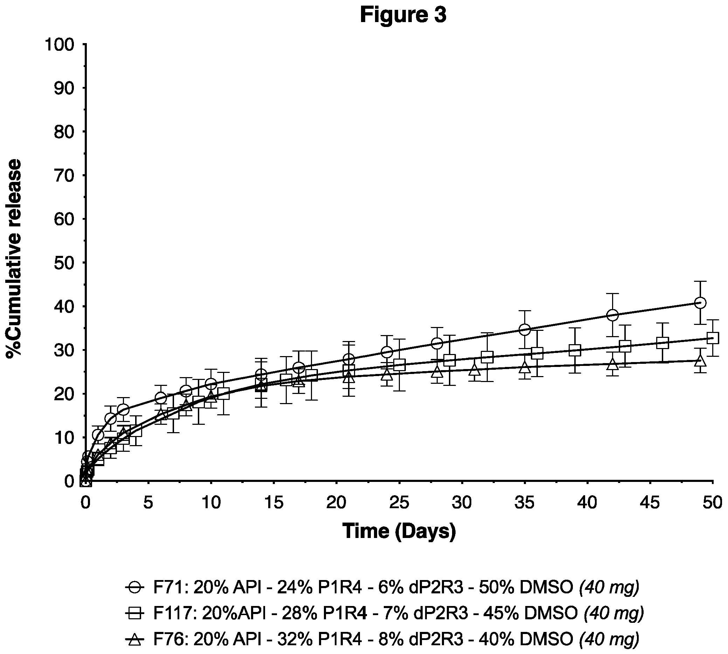

Figure 3 shows the *in vitro* cumulative release of tacrolimus over time from three different formulations (F71, F76 and F117). Formulations are loaded with P1R4 and dP2R3 (TB/DB = 4/1). The API loading is identical and fixed at 20% (w/w %) for all the formulations, but the copolymer content is different: 30, 35 or 40% (w/w %).

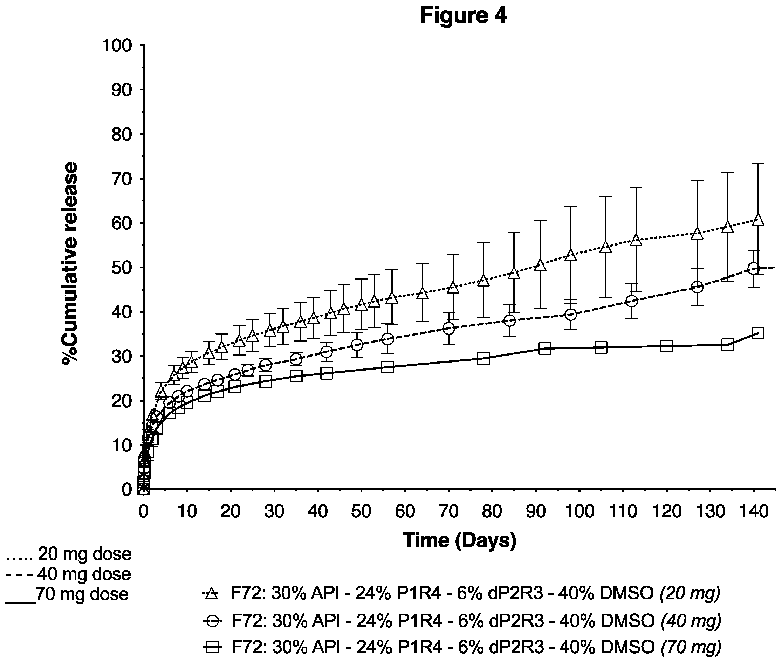

Figure 4 shows the *in vitro* cumulative release of tacrolimus over time from one formulation (F72) but different doses. Formulation is loaded with 30% (w/w %) of copolymers (P1R4/dP2R3 = 4/1) and 30% (w/w %) of API. The API dose was fixed at 20, 40 or 70 mg/depot.

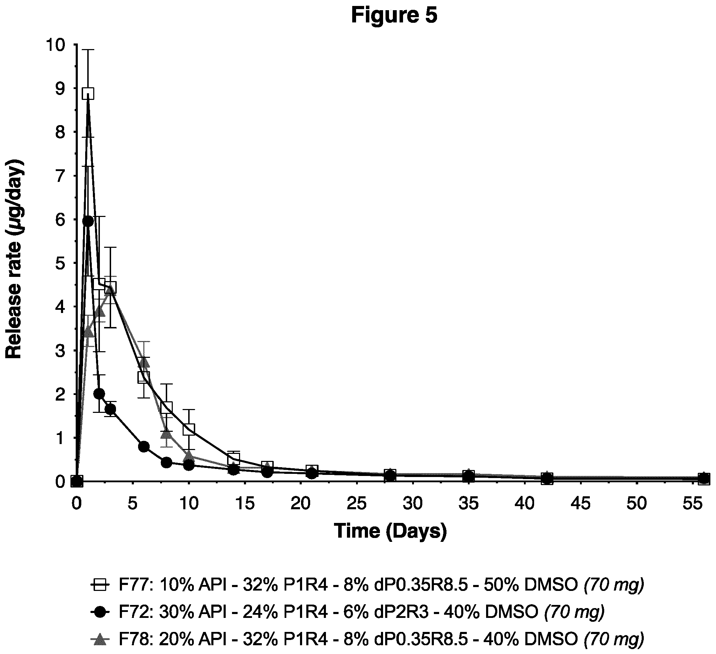

Figure 5 shows the *in vitro* release rate of tacrolimus over time from three different formulations. Formulations are loaded with P1R4 and different diblocks: dP0.35R8.5 or dP2R3 (TB/DB = 4/1) with the total copolymer content varying from 30 to 40% (w/w %). The API loadings are different: 10, 20 or 30% (w/w %), but the API dose was fixed at 70 mg for all the formulations. Dose is adjusted by varying the depot size.

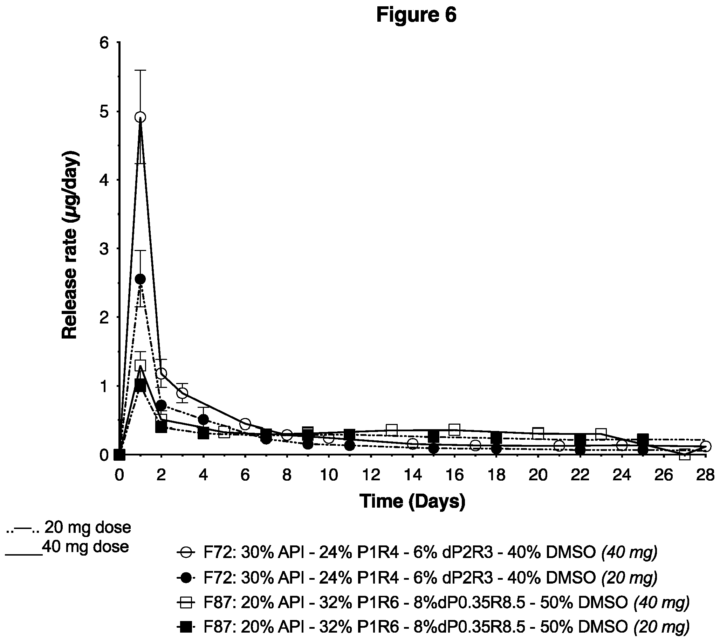

Figure 6 shows the *in vitro* release rate of tacrolimus over time from two formulations (F72 and F87) at different doses. Formulations are loaded with 30% (w/w %) of P1R4/dP2R3 or with 40% (w/w %) of P1R6/dP0.35R8.5 (TB/DB = 4/1). The API dose was fixed at 20 and 40 mg. Dose is adjusted by varying the depot size.

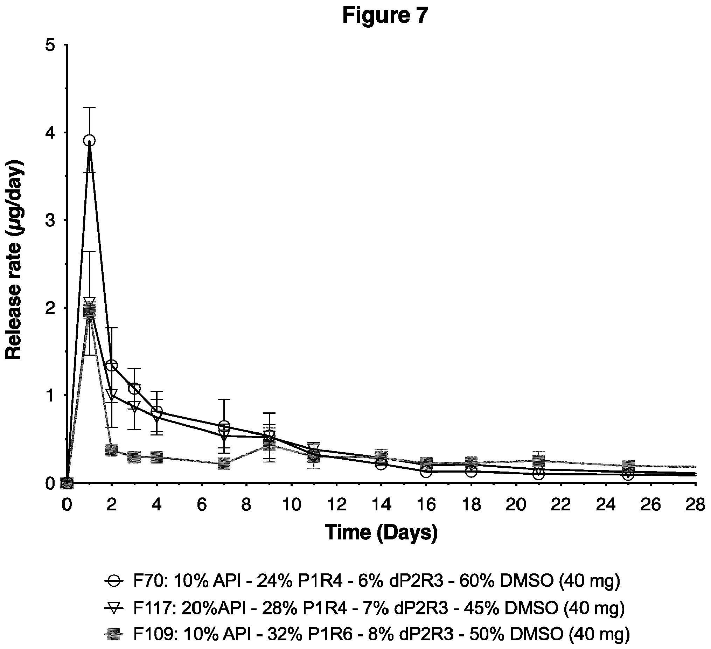

Figure 7 shows the *in vitro* release rate of tacrolimus over time from three different formulations (F70, F109 and F117). Formulations are loaded with P1R4 or P1R6 and dP2R3 (TB/DB = 4/1). The copolymer content and the API loading are different for all the formulations, but the API dose was fixed at 40 mg. Dose is adjusted by varying the depot size.

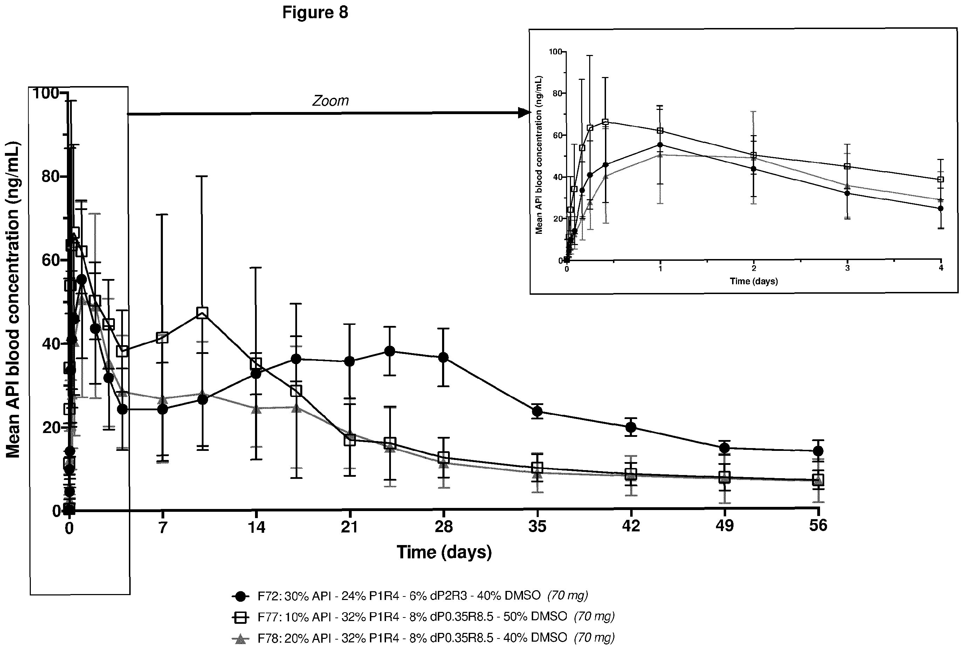

Figure 8 shows the average kinetic profiles of tacrolimus in blood after a single subcutaneous injection of a 70 mg API dose of formulations F72, F77 or F78.

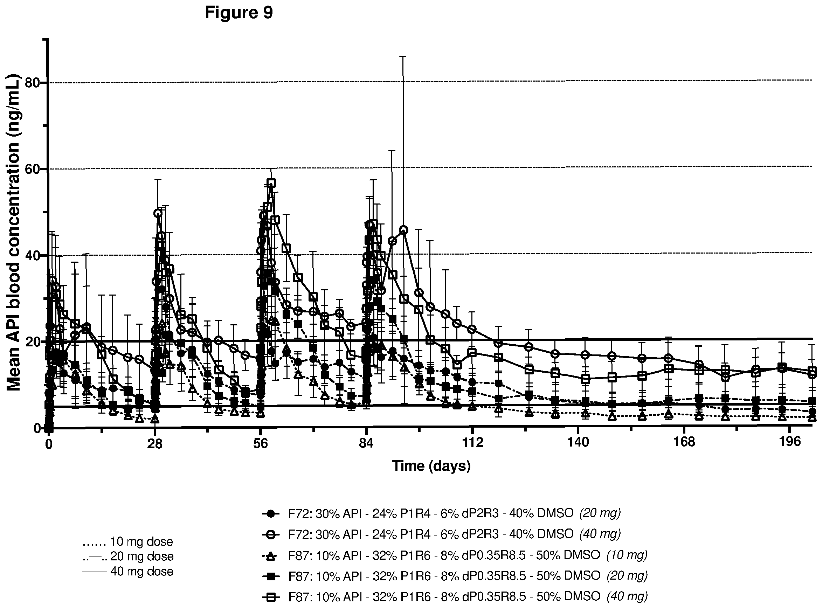

Figure 9 shows the average kinetic profiles of tacrolimus in blood over 203 days after 4 repeated monthly subcutaneous injections of formulations F72 or F87. Dose effect was evaluated by changing the volume of injection.

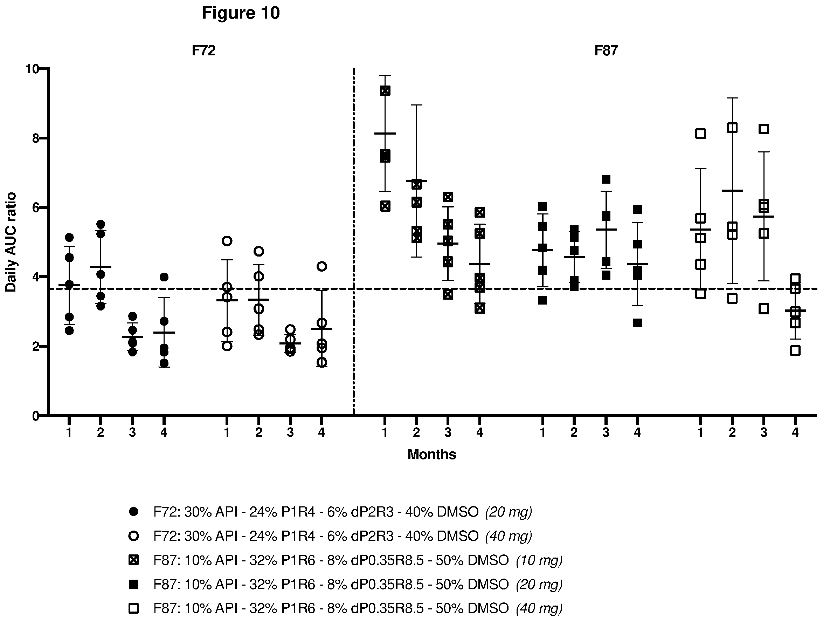

Figure 10 represents the mean and individuals daily AUC ratios estimated over a 28-day period after each subcutaneous injection of formulations F72 and F87 at different doses. The sustainability of tacrolimus blood concentrations within a narrow range is characterized by a mean daily AUC ratio close to or below the 3.65 threshold.

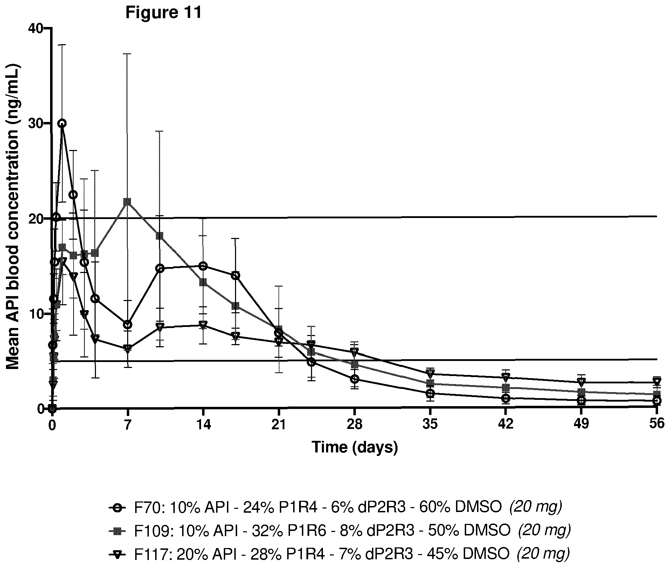

Figure 11 shows the mean blood concentration-time profiles of tacrolimus over 56 days after a single subcutaneous injection of formulations F70, F109 and F117.

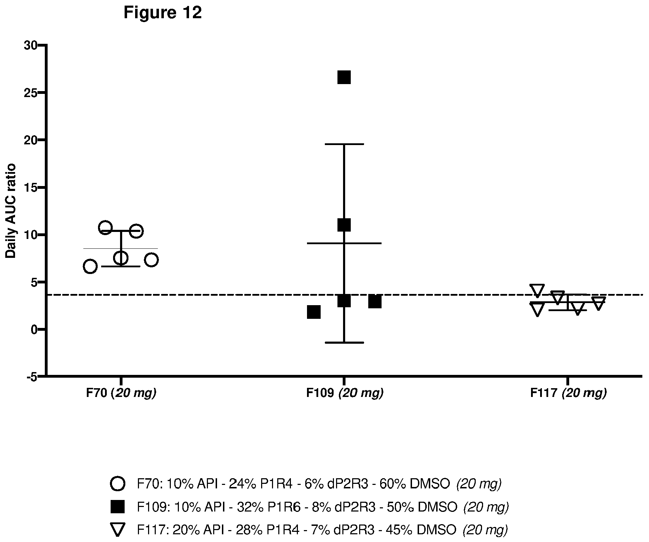

Figure 12 represents the mean and individual daily AUC ratios estimated over a 28-day period obtained after a single subcutaneous injection of formulations F70, F109 and F117. The sustainability of tacrolimus blood concentrations within a narrow range is characterized by a mean daily AUC ratio close to or below the 3.65 threshold.

### DETAILED DESCRIPTION

An aspect of the invention provides a pharmaceutical composition comprising
(a) a triblock copolymer having the formula:

   PLAᵥ-PEG_{w}-PLAₓ

   wherein v and x are the number of repeat units ranging from 1 to 3,000 and w is the number of repeat units ranging from 3 to 300 and v=x or v≠x in an amount of 22 to 34 w/w % of the total composition;
(b) a diblock copolymer having the formula:

   mPEG_{y}-PLA_{z}

   wherein y and z are the number of repeat units with y ranging from 2 to 250 and z ranging from 1 to 3,000 in an amount of 5 to 9 w/w% of the total composition;
(c) tacrolimus or a pharmaceutically acceptable salt thereof in an amount of 8 to 32 w/w % of the total composition; and
(d) organic solvent in an amount of 30 to 62 w/w % of the total composition.

A preferred embodiment provides a pharmaceutical composition comprising
(a) a triblock copolymer having the formula:

   PLAᵥ-PEG_{w}-PLAₓ

   wherein v and x are the number of repeat units ranging from 1 to 3,000 and w is the number of repeat units ranging from 3 to 300 and v=x or v≠x in an amount of 24 to 32 w/w % of the total composition;
(b) a diblock copolymer having the formula:

   mPEG_{y}-PLA_{z}

   wherein y and z are the number of repeat units with y ranging from 2 to 250 and z ranging from 1 to 3,000 in an amount of 6 to 8 w/w % of the total composition;
(c) tacrolimus or a pharmaceutically acceptable salt thereof in an amount of 10 to 30 w/w % of the total composition; and
(d) organic solvent in an amount of 30 to 60 w/w % of the total composition.

The triblock and diblock copolymers used in the present invention are "bioresorbable" which means that the block copolymers undergo hydrolytic cleavage *in vivo* to form their constituent (m)PEG and oligomers or monomers derived from the polyester block. For example, PLA undergoes hydrolysis to form lactic acid. The result of the hydrolysis process leads to a progressive mass loss of the depot and ultimately to its disappearance.

As used herein "repeat units" are the fundamental recurring units of a polymer.

As used herein "polyethylene glycol", as abbreviated PEG throughout the application, is sometimes referred to as poly(ethylene oxide) or poly(oxyethylene) and the terms are used interchangeably in the present invention. mPEG is methoxy polyethylene glycol.

The abbreviation of "PLA" refers to poly(lactic acid).

The copolymers have been named as follows:
The triblock copolymers described herein were labelled PxRy where x represents the molecular weight of the PEG chain in kDa and y is the lactic acid/ethylene oxide (LA/EO) molar ratio and allows the calculation of the PLA chain length within the copolymer.

The diblock copolymers described herein were labelled dPxRy where x represents the molecular weight of the mPEG chain in kDa and y is the lactic acid/ethylene oxide (LA/EO) molar ratio.

The molecular weight of the PEG chain, also referred to as the PEG repeat unit, namely -(CH₂CH₂O)ₙ- where n is an integer, is measured by gel permeation chromatography (GPC), typically with a polystyrene standard. The molecular weight measured is number average molecular weight (Mn).

General formulae for the diblock and triblock copolymers are set out below:
Diblock Copolymers (DB) 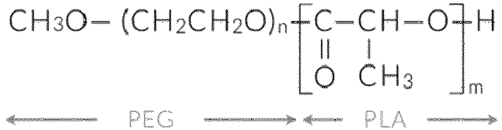
Triblock Copolymers (TB)

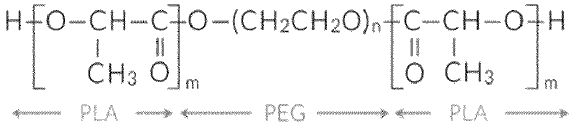

In the present specification the composition comprising the diblock copolymer, triblock copolymer and solvent, i.e. the pharmaceutical composition minus the tacrolimus may be referred to as the "vehicle".

The organic solvent may be selected from the group of: benzyl alcohol, benzyl benzoate, dimethyl isosorbide (DMI), dimethyl sulfoxide (DMSO), ethyl acetate, ethyl benzoate, ethyl lactate, glycerol formal, methyl ethyl ketone, methyl isobutyl ketone, N-ethyl-2-pyrrolidone, N-methyl-2-pyrrolidinone(NMP), pyrrolidone-2, tetraglycol, triacetin, tributyrin, tripropionin, glycofurol, and mixtures thereof. In one embodiment DMSO, NMP, tripropionin or mixtures thereof can be used as solvents. Preferably the organic solvent is dimethyl sulfoxide.

The "injectability" of a formulation, as used herein, is defined by the force needed in Newtons (N) to inject a formulation using pre-determined parameters. These parameters include injection speed, injection volume, injection duration, syringe type or needle type and the like. These parameters may vary based on specific formulation, or the desired method of administration such as subcutaneous, perineural, intra articular and the like. They may be adjusted to be able to observe the differences and fluctuations between the formulations. The injectability must be kept low such that the formulation can be easily administered by a qualified healthcare professional in an acceptable timeframe. An acceptable injectability value may be from 10 N to 20N. A non-optimal injectability may be greater than 20 N but less than 30 N. Injectability may be measured using a texturometer, preferably a Lloyd Instruments FT plus texturometer, using the following analytical conditions: 500 µL of formulation are injected through a 1 mL Soft-ject syringe, using a 21G 1" Terumo needle with a 550 µL/min flow rate at 23°C.

"Viscosity," by definition and as used herein, is a measure of a fluid's resistance to flow and gradual deformation by shear stress or tensile strength. It describes the internal friction of a moving fluid. For liquids, it corresponds to the informal concept of "thickness". By 'dynamic viscosity" is meant a measure of the resistance to flow of a fluid under an applied force. The dynamic velocity can range from 100 mPa.s to 3000 mPa.s. Preferably the composition has a dynamic viscosity of from about 1000 to about 2000 mPa.s, optionally from about 1500 to about 1700 mPa.s.

Dynamic viscosity is determined using an Anton Paar Rheometer equipped with cone plate measuring system. Typically, 700 µL of studied formulation are placed on the measuring plate. The temperature is controlled at +25°C. The measuring system used is a cone plate with a diameter of 50 mm and a cone angle of 1 degree (CP50-1). The working range is from 10 to 1000 s⁻¹, preferably 100 s⁻¹. Formulations are placed at the center of the thermo-regulated measuring plate using a positive displacement pipette. The measuring system is lowered down and a 0.104 mm gap is left between the measuring system and the measuring plate. 21 viscosity measurement points are determined across the 10 to 1000 s⁻¹ shear rate range. Given values are the ones obtained at 100 s⁻¹.

In a further aspect, the present invention provides a pharmaceutical composition for use in the therapeutic suppression of the immune system of a subject, wherein the use comprises administering the pharmaceutical composition to the subject, said composition comprising:
(a) a triblock copolymer having the formula:

   PLAᵥ-PEG_{w}-PLAₓ

   wherein v and x are the number of repeat units ranging from 1 to 3,000 and w is the number of repeat units ranging from 3 to 300 and v=x or v≠x in an amount of 22 to 34 w/w % of the total composition;
(b) a diblock copolymer having the formula:

   mPEG_{y}-PLA_{z}

   wherein y and z are the number of repeat units with y ranging from 2 to 250 and z ranging from 1 to 3,000 in an amount of 5 to 9 w/w % of the total composition;
(c) tacrolimus or a pharmaceutically acceptable salt thereof in an amount of 8 to 32 w/w % of the total composition; and
(d) organic solvent in an amount of 30 to 62 w/w % of the total composition.

In a preferred embodiment the invention provides a pharmaceutical composition for use in the therapeutic suppression of the immune system of a subject, wherein the use comprises administering the pharmaceutical composition to the subject, said composition comprising:
(a) a triblock copolymer having the formula:

   PLAᵥ-PEG_{w}-PLAₓ

   wherein v and x are the number of repeat units ranging from 1 to 3,000 and w is the number of repeat units ranging from 3 to 300 and v=x or v≠x in an amount of 24 to 32 w/w % of the total composition;
(b) a diblock copolymer having the formula:

   mPEG_{y}-PLA_{z}

   wherein y and z are the number of repeat units with y ranging from 2 to 250 and z ranging from 1 to 3,000 in an amount of 6 to 8 w/w % of the total composition;
(c) tacrolimus or a pharmaceutically acceptable salt thereof in an amount of 10 to 30 w/w % of the total composition; and
(d) organic solvent in an amount of 30 to 60 w/w % of the total composition.

Therapeutic suppression of the immune system can also be referred to as use as an immunosuppressant.

In a preferred embodiment, for the triblock copolymer w is an integer from 20 to 25 and v and x are each an integer from 40 to 50, preferably wherein w is about 22 and v and x are each about 45; or wherein w is an integer from 20 to 25 and v and x are each an integer from 60 to 75, preferably wherein w is about 22 and v and x are each about 68.

Typically, for the triblock copolymer the molecular weight of the PEG repeat unit is 1 kDa, and the lactic acid/ethylene oxide molar ratio is 4 or 6.

In a preferred embodiment, for the diblock copolymer y is an integer from 7 to 10 and z is an integer from 60 to 85, preferably wherein y is about 8 and z is about 68; or wherein y is an integer from 43 to 47 and z is an integer from 129 to 141, preferably wherein y is about 45 and z is about 136.

Typically, for the diblock copolymer the molecular weight of the PEG repeat unit is 0.35 kDa and the lactic acid/ethylene oxide molar ratio is 8.5; or the molecular weight of the PEG repeat unit is 2kDa and the lactic acid/ethylene oxide molar ratio is 3.

In one embodiment of the composition or composition for use for the triblock copolymer the molecular weight of the PEG repeat unit is 1 kDa and the lactic acid/ethylene oxide molar ratio is 4.

In another embodiment of the composition or composition for use for the triblock copolymer the molecular weight of the PEG repeat unit is 1 kDa and the lactic acid/ethylene oxide molar ratio is 6.

In one embodiment of the composition or composition for use for the diblock copolymer the molecular weight of the PEG repeat unit is 0.35 kDa and the lactic acid/ethylene oxide molar ratio is 8.5.

In another embodiment of the composition or composition for use for the diblock copolymer the molecular weight of the PEG repeat unit is 2 kDa and the lactic acid/ethylene oxide molar ratio is 3.

A plurality of doses of the composition may be administered to the subject. This typically means single doses administered every 2 weeks, more preferably every 4 weeks or every month. The compositions are typically administered over the lifetime of the subject. This is particularly the case if the composition is administered to a subject who has received an organ transplant where continuous administration of the immunosuppressant is required to prevent organ rejection.

In preferred embodiments, the time period between administration of consecutive doses is at least 14 days, preferably 21 days, most preferably at least 28 days. The time period between administration of consecutive doses may be at least 35 days or at least 42 days. Typically the composition is administered to the subject every 2 weeks, or every 3 weeks, preferably every 4 weeks. The administration may be monthly.

Typically, the use comprises parenteral administration of the composition, preferably subcutaneous injection of the composition. Subcutaneous administration is understood to provide significant advantages over oral or intravenous modes of administration since it leads to a reduction in intra and inter-patient variability in tacrolimus absorption and efficacy. The composition may be injected using a standard needle and syringe. Alternatively, the composition may be injected using an injection device. The use of an injection device allows for the delivery of an accurate volume of the composition to the subject. This is particularly important for compositions having a viscosity above 1000 mPa.s⁻¹. By administering an accurate volume of the composition to the subject, the administered dose can be precisely controlled. Typically, the dose of tacrolimus administered to the subject is controlled by adjusting the volume of the composition administered to the subject.

Preferably the composition releases a therapeutic concentration of tacrolimus into the blood plasma of the subject for at least 14 days, preferably at least 21 days, most preferably at least 28 days after administration of the composition. Typically, the therapeutic concentration of tacrolimus in the blood plasma of the subject is 5 to 20 ng/mL. However, the concentration of tacrolimus can be higher than 20 ng/mL for a period of up to 48 hours during the treatment period. For example, there may be an initial "spike" in the tacrolimus concentration provided that this does not extend past 48 hours after administration, and that the target value of 5 to 20 ng/mL is substantially maintained. An advantage of the composition of the invention is that it allows controlled release of tacrolimus within a narrow concentration range (5 to 20 ng/mL) over an extended period. There is a narrow therapeutic window for tacrolimus treatment, and it is critical that administration of the composition does not lead to sudden changes in blood plasma tacrolimus concentration.

The inventors have found that the pharmaceutical composition of the present invention provides acceptable pharmacokinetic properties.

The "Area under the Curve" or AUC is the definite integral of a curve that describes the variation of a drug concentration in blood plasma as a function of time within a defined time-period. A skilled person knows how to determine the AUC which is a standard pharmacokinetic parameter. With some assumptions, the area under the curve is a parameter that is closely dependent on the drug amount that enters into the systemic circulation and on the ability that the system has to eliminate the drug (clearance). Therefore it can be used to measure the drug amount absorbed or the efficiency of physiological processes that characterize the drug elimination. Preferably, the daily AUC, namely the AUC for a 24 h period (daily AUC = AUC0-24h), is within the range of from about 130 to 475 ng.h/mL at steady state, that also corresponds to clinical efficacy in humans (Advagraf^{®}, Product Information). The area under the concentration vs. time curve (AUC) can be calculated using the linear trapezoidal method.

Typically the use comprises maintaining between administrations the daily AUC ratio, calculated as the ratio of the maximum daily AUC over the minimum daily AUC over a defined period, below a maximum value in the range of from about 3.60 to about 3.70, preferably below about 3.65 (ratio of 475/130).

In a preferred embodiment the composition comprises a dose concentration of tacrolimus of from about 115 to about 345 mg/mL, preferably about 230 mg/mL.

Typically the dose of tacrolimus is administered in a volume of from about 0.1 mL to about 2 mL, optionally from about 0.1 mL to about 1.5 mL, optionally from about 0.1 mL to about 1 mL, optionally from about 0.1 mL to about 0.5 mL. By adjusting the volume administered to the subject, the dose administered can be altered. This allows the dose administered to be easily tailored to individual patients.

The composition may be injected over a period of about 1 second to about 2 minutes, optionally about 1 second to 1 minute, optionally about about 1 second to about 30 seconds, optionally about 1 second to about 20 seconds, optionally about 1 second to about 10 seconds, optionally about 1 to about 5 seconds.

Most preferably, the use comprises prophylaxis and/or treatment of allograft rejection in a subject who has received an allograft. This may also be referred to as prophylaxis and/or treatment of organ transplant rejection in a subject who has received an organ transplant. A particularly preferred embodiment is a composition as defined above for use in the prophylaxis and/or treatment of kidney transplant rejection in a subject who has received a kidney transplant.

An allotransplant is the transplantation of cells, tissues, or organs to a recipient from a genetically non-identical donor of the same species. The transplant is called an allograft, allogeneic transplant, or homograft. Most human tissue, cell and organ transplants are allografts.

The allograft may be a transplanted organ, tissue or cells. Preferably, the allograft is a liver, kidney or heart, most preferably a kidney. An immune response against an allograft is termed rejection. The compositions of the invention may also be useful for the prophylaxis and/or treatment of xenograft rejection in a subject who has received a xenograft. A xenograft is a transplant of an organ, tissue or cells from a different species.

Rejection is an adaptive immune response via cellular immunity (mediated by killer T cells inducing apoptosis of target cells) as well as humoral immunity (mediated by activated B cells secreting antibody molecules), though the action is joined by components of innate immune response (phagocytes and soluble immune proteins). Tacrolimus is a potent immunosuppressant, i.e. it prevents and/or treats organ or tissue rejection by suppressing the immune system's response to the foreign organ, tissue or cells (the allograft or xenograft).

In an alternative embodiment, the use comprises prophylaxis and/or treatment of an autoimmune disease. A skilled person would be aware of autoimmune diseases or conditions that can be treated by the compositions of the invention. The use may comprise the prophylaxis and/or treatment of Lupus Nephritis.

In a preferred embodiment of the compositions of the invention, the weight ratio of the triblock copolymer to the diblock copolymer is about 4:1.

In a preferred embodiment, the composition comprises about 10 to 30 w/w % tacrolimus, optionally about 10 to 20 w/w % tacrolimus, optionally about 20 w/w % tacrolimus, optionally about 10 w/w % tacrolimus.

In a preferred embodiment the composition or composition for use comprises about 24 to 32 w/w % of the triblock copolymer, optionally 26 to 30 w/w % of the triblock copolymer, optionally about 27 to 29 w/w % of the triblock copolymer, optionally about 28 w/w% of the triblock copolymer, optionally about 24 w/w% of the triblock copolymer.

In a preferred embodiment the composition or composition for use comprises about 6 to 8 w/w% of the diblock copolymer, optionally about 7 to 8 w/w% of the diblock copolymer, optionally about 7 w/w% of the diblock copolymer, optionally about 6 w/w% of the diblock copolymer.

Typically the composition or composition for use comprises about 30 to 60 w/w%, optionally about 40 to 50 w/w%, optionally about 45 to 50 w/w%, optionally about 45 w/w% dimethyl sulfoxide, optionally about 60 w/w% dimethyl sulfoxide.

The composition or composition for use may have a dynamic viscosity of from about 200 to about 3000 mPa.s, from about 1000 to about 3000 mPa.s, from about 1000 mPa.s. to about 2000 mPa.s, preferably from about 1500 mPa.s to about 1700 mPa.s

The composition or composition for use may further comprise one or more pharmaceutically-acceptable excipients.

In a particularly preferred embodiment the composition or composition for use comprises 27-29 w/w % of the triblock copolymer, 6-8 w/w % of the diblock copolymer, 44-46 w/w % dimethyl sulfoxide, and 19-21 w/w % tacrolimus, and wherein for the triblock copolymer the molecular weight of the PEG repeat unit is 1 kDa and the lactic acid/ethylene oxide molar ratio is 4, and for the diblock copolymer the molecular weight of the PEG repeat unit is 2 kDa and the lactic acid/ethylene oxide molar ratio is 3.

In another particularly preferred embodiment the composition or composition for use comprises 23-25 w/w % of the triblock copolymer, 5-7 w/w % of the diblock copolymer, 59-61 w/w % dimethyl sulfoxide, and 9-11 w/w % tacrolimus, and wherein for the triblock copolymer the molecular weight of the PEG repeat unit is 1 kDa and the lactic acid/ethylene oxide molar ratio is 4, and for the diblock copolymer the molecular weight of the PEG repeat unit is 2 kDa and the lactic acid/ethylene oxide molar ratio is 3.

In a further aspect, the invention provides a method of therapeutic suppression of the immune system of a subject as described above, the method comprising the step of administering a composition as defined above to the subject. The wording "therapeutic suppression of the immune system" means suppression of the immune system which leads to the prophylaxis and/or treatment of an immune condition or disease, such as allograft rejection or autoimmune disease.

In a further aspect, the present invention provides a method of preventing or treating allograft rejection in a subject who has received an allograft as described above, the method comprising the step of administering a composition as defined above to the subject.

The composition may be administered to a human or non-human animal.

The composition or composition for use described above is typically suitable for forming a depot when injected into the body, i.e. an *"in situ* depot".

The compositions of the invention are administered via depot injection. The term "depot injection" is an injection of a flowing pharmaceutical composition, usually subcutaneous, intradermal or intramuscular that deposits a drug in a localized mass, such as a solid or semi-solid mass, called a "depot". The depots as defined herein are *in situ* forming upon injection. Thus, the formulations can be prepared as solutions or suspensions and can be injected into the body. In the present invention the composition is typically administered via subcutaneous injection and a depot is formed under the skin.

An *"in situ* depot" is a solid or semi-solid, localized mass formed by precipitation of the pharmaceutical composition after injection of the composition into the subject. The pharmaceutical composition comprises copolymers which are substantially insoluble in aqueous solution. Thus, when the pharmaceutical composition contacts the aqueous environment of the human or animal body, a phase inversion occurs causing the composition to change from a liquid to a solid, i.e. precipitation of the composition occurs, leading to formation of an *"in situ* depot".

An *"in situ* depot" can be clearly distinguished from hydrogel pharmaceutical formulations described in the prior art. Hydrogels have three-dimensional networks that are able to absorb large quantities of water. The polymers making up hydrogels are soluble in aqueous solution. By contrast, the polymers used in the present invention are substantially insoluble in aqueous solution. The pharmaceutical compositions of the invention are free of water, or substantially free of water. For example, the pharmaceutical compositions of the invention comprise less than 0.5% w/w water.

Wherever embodiments are described with the language "comprising," otherwise analogous embodiments described in terms of "consisting of" and/or "consisting essentially of" are included.

All of the references cited in this disclosure are hereby incorporated by reference in their entireties. In addition, any manufacturers' instructions or catalogues for any products cited or mentioned herein are incorporated by reference. Documents incorporated by reference into this text, or any teachings therein, can be used in the practice of the present invention. Documents incorporated by reference into this text are not admitted to be prior art.

### List of abbreviations:

- API: Active Pharmaceutical Ingredient
- AUC: Area under the curve within a certain time-span
- C_{ac}: Average concentration per partial area (dividing the partial area by the interval time)
- Cₗₐₛₜ: Last measurable blood plasma concentration
- Cₘₐₓ: Maximum blood plasma concentration
- DB: Diblock (mPEG-PLA)
- DMSO: Dimethyl sulfoxide
- EO: Ethylene oxide
- g: Gram
- G: Gauge
- GPC: Gel permeation chromatography
- UPLC: Ultra-performance liquid chromatography
- IV: Intrinsic Viscosity
- kDa: KiloDalton
- LA: Lactic acid
- mg: Milligram
- mg/mL: Milligram per millilitre
- mPEG: methoxy-Polyethylene Glycol
- mPEG-PLA: methoxy-Polyethylene Glycol -poly(lactic acid)
- PEG: Polyethylene Glycol
- PLA-PEG-PLA: Poly(lactic acid)-Poly(ethylene glycol)-poly(lactic acid)
- PLA: Poly(lactic acid)
- t_{1/2}: Elimination half-life
- TB: Triblock (PLA-PEG-PLA)
- tₗₐₛₜ: Time point of last measurable blood concentration
- tₘₐₓ: Time point at which maximum blood concentration was reached

### EXAMPLES

### Example 1: Materials

### Linear block copolymers synthesis

Copolymers were synthesized according to the method described in the US6,350,812, incorporated herein by reference, with minor modifications. Typically, the necessary amount of PEG (gives the triblock co-polymer) or methoxy-PEG (gives the diblock copolymer) was heated at 65°C and dried under vacuum for 2 hours in a reactor vessel. DL-lactide (corresponding to the targeted LA/EO molar ratio) and zinc lactate (1/1000 of amount of lactide) were added. The reaction mixture was first dehydrated by three short vacuum/N2 cycles. The reaction mixture was heated at 140°C and rapidly degassed under vacuum. The reaction was conducted for four days at 140°C under constant nitrogen flow (0.2 bar). The reaction was cooled to room temperature and its content was dissolved in acetone and then subjected to precipitation with ethanol. The product obtained was subsequently dried under reduced pressure. The triblock PLA-PEG-PLA polymers described herein are labelled PxRy, where x represent the size of the PEG chain in kDa and y is the LA/EO molar ratio. The diblock mPEG-PLA polymers described herein are labelled dPxRy where x represents the size of the PEG chain in kDa and y is the LA/EO molar ratio.

The product obtained was characterized by ¹H NMR for its residual lactide content and for the determination of the R ratio.

¹H NMR spectroscopy was performed using a Brucker Advance 300 MHz spectrometer. For all ¹H NMR spectrograms, MestReNova software was used for the integration of peaks and their analyses. Chemical shifts were referenced to the δ = 7.26 ppm solvent value of CDCl3.
For the determination of the R ratio, which describes the ratio between lactic acid units over ethylene oxide units (LA/EO), all peaks were integrated separately. The intensity of the signal (integration value) is directly proportional to the number of hydrogens that constitutes the signal. To determine the R ratio (LA/EO ratio), the integration values need to be homogenous and representative of the same number of protons (e.g. all signal values are determined for 1H). One characteristic peak of PLA and one of PEG are then used to determine the LA/EO ratio. This method is valid for molecular weights of PEGs above 1000 g/mol where the signal obtained for the polymer end-functions can be neglected.

### Example 2: In vitro release tests

### Formulation preparation:

In an empty and tared glass vial, required copolymer amounts were weighed. The glass vial was tared again. An accurate DMSO mass was added using a Pasteur pipette. Vehicles (copolymer + solvent) were then placed on a roller mixer at room temperature (RT) for 6 to 7 hours until complete copolymer dissolution. New appropriate glass vial was then tared, and the required API amount was weighed. With a 10 mL syringe the right amount of vehicle was then add on the top of the API powder. Vials were protected from the light with aluminum. The formulations were then placed overnight at room temperature on a roller mixer.

### In vitro release set-up:

Depending on the initial Tacrolimus content in the formulations and the targeted dose, the injection volume was determined. The appropriate amount of formulation was then withdrawn from the corresponding glass vial previously vortexed, into a 0.5 mL Codan syringe. The syringe was cleaned, tared and the formulation was directly injected from the syringe without needle, into a 150 mL prefilled Erlenmeyer containing 50 mL of release buffer. The buffer used is phosphate-buffered saline (PBS) pH 7.4, with 1% of Brij^{®}35. Upon injection, the solvent diffused away from the formulation and the remaining polymer forms an in-situ depot within the aqueous environment. Once precipitation and depot formation had occurred, the depot was separated from the syringe using a scalpel. The syringe was weighed back to determine the accurate depot mass.

The tacrolimus incorporated into the polymer solution was encapsulated within the polymer matrix as it solidifies.

Once all depots were formed, glass vials were maintained under constant shaking at 74 rpm at 37°C.

IVR was analyzed following the steps detailed below:

### IVR sampling and preparation of IVR samples for API quantification

At each desired time point, a sufficient amount of buffer was withdrawn for analysis from the 150 mL Erlenmeyer before total buffer refreshment. 1 mL of each sample was filtered through a 0.2 µm hydrophilic filter into a 1 mL HPLC glass vial. The rest of the medium was discarded and 50 mL of fresh buffer were added to the glass vial. Sink conditions were maintained during the full duration of the study. API contents in released buffer were determined using UPLC. The amount of API released from the formulation was calculated from a calibration curve where the concentration of Tacrolimus ranges between 1 and 200 µg/ml.

Results of *in vitro* release tests were used to select the candidates studied during *in vivo* studies. Studied formulations are presented in Table 1 below.

**Table 1**

| **Formulation** | **API** | | **TB copolymer** | | | **dB copolymer** | | | **Solvent** | |
|---|---|---|---|---|---|---|---|---|---|---|
| Number | Name | % (w/w) | Code | Experimental Ratio LA/EO | % (w/w) | Code | Experimental Ratio LA/EO | % (w/w) | Name | % (w/w) |
| 70 | Tacrolimus | 10.00 | P1R4 | 4.0 | 24.00 | dP2R3 | 3.0 | 6.00 | DMSO | 60.00 |
| 71 | Tacrolimus | 20.00 | P1R4 | 3.6 | 24.00 | dP2R3 | 2.9 | 6.00 | DMSO | 50.00 |
| 72 | Tacrolimus | 30.00 | P1R4 | 3.6 | 24.00 | dP2R3 | 2.9 | 6.00 | DMSO | 40.00 |
| 76 | Tacrolimus | 20.00 | P1R4 | 3.6 | 32.00 | dP2R3 | 2.9 | 8.00 | DMSO | 40.00 |
| 77 | Tacrolimus | 10.00 | P1R4 | 3.6 | 32.00 | dP0.35R8.5 | 8.8 | 8.00 | DMSO | 50.00 |
| 78 | Tacrolimus | 20.00 | P1R4 | 3.6 | 32.00 | dP0.35R8.5 | 8.8 | 8.00 | DMSO | 40.00 |
| 87 | Tacrolimus | 10.00 | P1R6 | 5.5 | 32.00 | dP0.35R8.5 | 10.1 | 8.00 | DMSO | 50.00 |
| 88 | Tacrolimus | 20.00 | P1R6 | 5.5 | 32.00 | dP0.35R8.5 | 10.1 | 8.00 | DMSO | 40.00 |
| 109 | Tacrolimus | 10.00 | P1R6 | 6.5 | 32.00 | dP2R3 | 3.0 | 8.00 | DMSO | 50.00 |
| 117 | Tacrolimus | 20.00 | P1R4 | 4.0 | 28.00 | dP2R3 | 3.0 | 7.00 | DMSO | 45.00 |

### Example 3 : Injectability

Injectability analyses were performed using a Lloyd Instruments FT plus texturometer following the procedure described below:
Formulations were vortexed for 15 seconds. 500 µL of formulation were withdrawn using a 1 mL Soft-ject syringe without needle. Air bubbles were removed to avoid any interference during the injectability measurement. A 21G 1" Terumo needle was then mounted on the syringe. The syringe was placed onto the texturometer. The flow rate was fixed at 500 µL/min. Injection of the formulation started at fixed speed. The injection device (i.e. syringe + needle) was changed for each replicate.

The average force in Newton (N) necessary to inject each replicate was calculated using texturometer software. The injectability analyses were performed in triplicates when sufficient amount was available.

**Table 2**

| **Formulation** | | **API** | | **TB copolymer** | | **dB copolymer** | | **Solvent** | | **Injectability** | | |
|---|---|---|---|---|---|---|---|---|---|---|---|---|
| Number | Batch number | Name | % (w/w) | Code | % (w/w) | Code | % (w/w) | Name | % (w/w) | Replicate number | Force (N) | SD |
| F70 | 6 | Tacrolimus | 10.00 | P1R4 | 24.00 | dP2R3 | 6.00 | DMSO | 60.00 | 3 | 3.1 | 0.3 |
| F72 | 3 | Tacrolimus | 30.00 | P1R4 | 24.00 | dP2R3 | 6.00 | DMSO | 40.00 | 2 | 22.7 | 0.9 |
| F77 | 2 | Tacrolimus | 10.00 | P1R4 | 32.00 | dP0.35R8.5 | 8.00 | DMSO | 50.00 | 2 | 8.4 | 2.2 |
| F78 | 2 | Tacrolimus | 20.00 | P1R4 | 32.00 | dP0.35R8.5 | 8.00 | DMSO | 40.00 | 2 | 17.0 | 3.2 |
| F87 | 2 | Tacrolimus | 10.00 | P1R6 | 32.00 | dP0.35R8.5 | 8.00 | DMSO | 50.00 | 2 | 10.6 | 0.1 |
| F88 | 2 | Tacrolimus | 20.00 | P1R6 | 32.00 | dP0.35R8.5 | 8.00 | DMSO | 40.00 | 2 | 48.9 | 2.8 |
| F109 | 2 | Tacrolimus | 10.00 | P1R6 | 32.00 | dP2R3 | 8.00 | DMSO | 50.00 | 3 | 18.2 | 0.5 |
| F117 | 2 | Tacrolimus | 20.00 | P1R4 | 28.00 | dP2R3 | 7.00 | DMSO | 45.00 | 3 | 14.5 | 0.2 |

### Example 4: Dynamic viscosity analysis

Dynamic viscosity analysis was performed using an Anton Paar Rheometer equipped with cone plate measuring system, with the following analytical conditions:
- Measuring system: cone plate of 50 mm diameter and cone angle of 1 degree (CP50). CP50 is better appropriate to obtain precise values for formulations showing low viscosity.
- Working range: from 10 to 1000 mPa.s.
- Temperature controlled at 25°C.
- Amount of formulation: 0.7 mL.

The formulation was vortexed for 10 s before analysis. The appropriate amount of sample was placed at the center of the thermo-regulated measuring plate using a spatula. The measuring system was lowered down and a 0.104 mm gap was left between the measuring system and the measuring plate. Twenty-one viscosity measurements points were determined across the 10 to 1000 s⁻¹ shear rate (10 points per decade).

Viscosity data correspond to those calculated at a shear rate of 100 s⁻¹, which is an average value of the curve plateau. The dynamic viscosity analyses were performed in triplicates when sufficient amount was available.

**Table 3**

| **Formulation** | | **API** | | **TB copolymer** | | **dB copolymer** | | **Solvent** | | **Viscosity** | | |
|---|---|---|---|---|---|---|---|---|---|---|---|---|
| Number | Batch number | Name | % (w/w) | Code | % (w/w) | Code | % (w/w) | Name | % (w/w) | Replicate number | Dynamic Viscosity (mP.s) | SD |
| F70 | 6 | Tacrolimus | 10.00 | P1R4 | 24.00 | dP2R3 | 6.00 | DMSO | 60.00 | 3 | 213.2 | 13.8 |
| F71 | 2 | Tacrolimus | 20.00 | P1R4 | 24.00 | dP2R3 | 6.00 | DMSO | 50.00 | 2 | 566.8 | 1.4 |
| F72 | 2 | Tacrolimus | 30.00 | P1R4 | 24.00 | dP2R3 | 6.00 | DMSO | 40.00 | 2 | 2759.1 | 18.9 |
| | 3 | | | | | | | | | 2 | 2751.7 | 12.9 |
| F76 | 1 | Tacrolimus | 20.00 | P1R4 | 32.00 | dP2R3 | 8.00 | DMSO | 40.00 | 2 | 3523.6 | 61.0 |
| F77 | 2 | Tacrolimus | 10.00 | P1R4 | 32.00 | dP0.35R8.5 | 8.00 | DMSO | 50.00 | 2 | 670.6 | 5.5 |
| F78 | 1 | Tacrolimus | 20.00 | P1R4 | 32.00 | dP0.35R8.5 | 8.00 | DMSO | 40.00 | 2 | 2902.1 | 26.7 |
| | 2 | | | | | | | | | 2 | 2243.7 | 19.2 |
| F87 | 2 | Tacrolimus | 10.00 | P1R6 | 32.00 | dP0.35R8.5 | 8.00 | DMSO | 50.00 | 2 | 1142.1 | 5.0 |
| F88 | 2 | Tacrolimus | 20.00 | P1R6 | 32.00 | dP0.35R8.5 | 8.00 | DMSO | 40.00 | 2 | 5486.6 | 84.2 |
| F109 | 2 | Tacrolimus | 10.00 | P1R6 | 32.00 | dP2R3 | 8.00 | DMSO | 50.00 | 3 | 1878.3 | 4.8 |
| F117 | 2 | Tacrolimus | 20.00 | P1R4 | 28.00 | dP2R3 | 7.00 | DMSO | 45.00 | 3 | 1592.7 | 10.0 |

### Example 5: Pharmacokinetic (PK) study:

Table 4 summarize the design of the different PK studies.

**Table 4: PKs experimental Design**

| **PK number** | **Test Item** | **API dose level (mg/animal)** | **Dose Volume (mL/animal)** | **Dose Concentration (mg/mL)** | **Needle** | **Injection duration (sec)** | **Dose Route** | **Number of Animals** |
|---|---|---|---|---|---|---|---|---|
| 1 | Tac solution | 2.5 | 12.5 | 0.2 | 23G | - | IV | 5 |
| | Tac solution | 2.5 | 12.5 | 0.2 | 21G | - | SC | 5 |
| | F77 | 70 | 0.610 | 100 | 21G | 15-20 | SC | 5 |
| | F78 | 70 | 0.300 | 200 | 19G | 10-15 | SC | 5 |
| | F72 | 70 | 0.200 | 300 | 19G | 5-10 | SC | 5 |
| 2 | F72 | 20 | 0.060 | 300 | 19G | 1-3 | SC | 5 |
| | | 40 | 0.120 | 300 | | 3-5 | SC | 5 |
| | F87 | 10 | 0.090 | 100 | 21G | 1-3 | SC | 5 |
| | | 20 | 0.175 | 100 | | 3-5 | SC | 5 |
| | | 40 | 0.350 | 100 | | 5-10 | SC | 5 |
| 3 | F70 | 20 | 0.175 | 100 | 21G | 3-5 | SC | 5 |
| | F109 | 20 | 0.175 | 100 | 21G | 3-5 | SC | 5 |
| | F117 | 20 | 0.090 | 200 | 21G | 1-3 | SC | 5 |

### PK1: A 2-Month Pharmacokinetic Study of Different Sustain-Release Formulations of Tacrolimus after Subcutaneous Administration and Subcutaneous Bioavailability Study of Tacrolimus in Female Göttingen Minipig

### Evaluation of the absolute bioavailability of Tacrolimus

*In vivo* detailed set-up procedure:
0.2 mg/mL Tacrolimus solution (Tacrolimus dissolved in vehicle containing 0.8% Kolliphor EL, 3.2% Ethanol 99.9% and 96% Saline solution) was tested in subcutaneous bioavailability study in female Göttingen minipig of 12-14 months old and with a weight between 20 and 30 kg. The dose volume for each animal was 12.5 mL corresponding to a unique dose of 2.5 mg/animal. Solution was administered:
- intravenously into the ear vein using 20 mL polypropylene syringe and 23G butterfly needle.
- subcutaneously in a skin fold caudal to the axillar region/shoulder using 20 mL polypropylene syringe and 21G needle.

Blood samples were collected into EDTA tubes at predose and at different time points:
- Intravenous dosing: 5 min (0.083 h), 15 min (0.25 h), 30 min (0.5 h), and 1, 2, 6, 24, 48, 72 and 96 h after end of dosing.
- Subcutaneous dosing: 30 min (0.5 h), and 1, 2, 4, 6, 10, 24, 48, 72 and 96 h after end of dosing.

Whole blood samples were analyzed to determine the concentration of the test item using a qualified analytical procedure.

### 2-month pharmacokinetic study of sustain-release formulations

*In vivo* detailed set-up procedure:
Three Tacrolimus formulations were tested in a pharmacokinetic study in female Göttingen minipig of 12-14 months old and with a weight between 20 and 30 kg. Drug products containing 70 mg of Tacrolimus were subcutaneously administered in a skin fold caudal to the axillar region/shoulder of the minipig using 1 mL Soft Ject^{®} syringes and 21G or 19G needles. Injected formulations volumes were fixed at 610 µL, 200 µL or 300 µL depending on the initial Tacrolimus content in the formulations. Blood samples were collected into EDTA tubes at predose and at different time points: T0.5h, T1h, T2h, T4h, T6h, T10h, T24h (Day 1), T48h (Day 2), T72h (Day 3), T96h (Day 4), T168h (Day 7), T240h (Day 10), T336h (Day 14), T408h (Day 17), T504h (Day 21), T576h (Day 24), T672h (Day 28), T840h (Day 35), T1008h (Day 42), T1176h (Day 49), T1344h (Day 56). Whole blood samples were analyzed to determine the concentration of the test item using a qualified analytical procedure.

Results are presented in figure 8 and table 5 summarizes corresponding PK parameters of interest.

**Table 5: Average ± SD PK1 parameters of Tacrolimus in Whole Blood after Single Subcutaneous Administration of 70 mg/animal F77, F78 and F72 (Pharmacokinetic Phase)**

| **Parameters** | **IV** | **SC** | **F77** | **F78** | **F72** |
|---|---|---|---|---|---|
| **Actual Dose Level (mg/animal)** | 2.47 ± 0.0267 | 2.51 ± 0.00799 | 71.3 ± 0.512 | 70.8 ± 1.25 | 72.0 ± 0.424 |
| **Actual Dose Level (mg/kg)** | 0.103 ± 0.00709 | 0.103 ± 0.00490 | 3.02 ± 0.307 | 2.74 ± 0.145 | 2.79 ± 0.233 |
| **Tₗₐₛₜ(h)** | 95.7 (95.7-96.1)^{$} | 96 (95.9-96.1)^{$} | 1343 | 1343 (1343-1344)^{$} | 1344 (1343-1344)^{$} |
| **Tₘₐₓ(h)** | - | 4 (1-6)^{$} | 9.88 (6-240)^{$} | 24 (23.9-48.1)^{$} | 23.2 (10.1-48.2)^{$} |
| **Cₘₐₓ (ng/mL)** | - | 80.2 ± 2.63 | 82.3 ± 24.0 | 54.0 ± 24.1 | 58.4 ± 17.8 |
| **Cₘₐₓ/D1 (kg.ng/mL/mg)** | - | 779 ± 30.8 | 26.9 ± 6.21 | 19.9 ± 9.37 | 21.2 ± 7.57 |
| **Cₗₐₛₜ(ng/mL)** | 4.22 ± 2.08 | 2.89 ± 0.759 | 6.73 ± 2.25 | 6.55 ± 5.13 | 13.6 ± 2.55 |
| **AUCₗₐₛₜ(h.ng/mL)** | 1850 ± 224 | 1760 ± 169 | 27800 ± 11000 | 22000 ± 10300 | 36200 ± 5220 |
| **AUCₗₐₛₜ/D1 (h.kg.ng/mL/mg)** | 17900 ± 1620 | 17100 ± 1090 | 9250 ± 3550 | 8090 ± 3960 | 13100 ± 2640 |
| **AUC_{∞}(h.ng/mL)** | 2040 ± 258 | 1870 ± 189 | 32900 ± 15400 | 22800 ± 6980 | 46600 ± 1000 |
| **AUC_{∞}/D1 (h.kg.ng/mL/mg)** | 19700 ± 1750 | 18100 ± 1150 | 11300 ± 4700 | 8250 ± 3030 | 17600 ± 1230 |
| **t_{½}(h)** | 31.5 ± 1.62 | 25.7 ± 2.25 | 644 ± 210 | 644 ± 210 | 644 ± 210 |
| **Δ between Cₘₐₓ and Cₗₐₛₜ (%)** | - | - | 91.0 ± 4.30 | 91.0 ± 4.30 | 91.0 ± 4.30 |

| | | | | | |
|---|---|---|---|---|---|
| ^{$}: median (range); /D1: dose-normalized by actual dose per kg; - : not applicable. | | | | | |

After intravenous administration, Tacrolimus was slowly cleared and highly distributed compound. Following subcutaneous administration, the Cₘₐₓ was reached at 4 hours and the average subcutaneous bioavailability of Tacrolimus was 92.4%.

Following subcutaneous administration of 70 mg/animal of F78, F77 and F72, all animals were exposed to Tacrolimus over 2 months. Its absorption was slow with the Cₘₐₓ reached between 10h and 24h. The burst was under control with the LAI formulations and the lowest Cₘₐₓ were observed with F72 and F78. These bursts are under control compared to the dose-normalized Cₘₐₓ obtained after the SC administration of the immediate release compound (779 kg·ng/mL/mg). The highest overall exposure and the lowest inter-variability were observed with F72. Finally, the difference between Cₘₐₓ and Cₗₐₛₜ was the lowest with F72.

### PK2: A Pharmacokinetic Study of Different Sustain-Release Formulations of Tacrolimus after 4 Monthly Repeated Administrations by the Subcutaneous Route in Female Göttingen Minipig with a 3-Month Follow-up Period.

*In vivo* detailed set-up procedure:
Two Tacrolimus formulations were tested in a pharmacokinetic study in female Göttingen minipig of 11-12 months old and with a weight between 22 and 25 kg. Three different Tacrolimus doses were tested: 10 mg, 20 mg and 40 mg. Drug products containing 10 mg, 20mg or 40 mg of Tacrolimus were subcutaneously administered in a skin fold caudal to the axillar region/shoulder of the minipig using 1 mL Soft Ject^{®} syringes and 21G or 19G needles. Injected formulations volumes were fixed between 60 and 350 µL depending on the initial Tacrolimus content in the formulations and the targeted dose. 4 monthly repeated administrations were made followed by a 3-month follow-up period. Blood samples were collected into EDTA tubes at predose and at different time points:
- After each injection (on Day 1, 29, 57 and 85): T2h, T4h, T6h, T10h, T24h, T48h, T72h, T96h, T168h, T240h, T336h, T408h, T504h, T576h, T672h. Whole blood samples were analyzed for concentration of test item using a qualified analytical procedure.
- During the 3-month follow-up period: Once a week until the end of the study (4 time points/ month), on Days 120, 128, 135, 150, 158, 165, 173, 180, 188, 195 and 203.

Whole blood samples were analyzed to determine the concentration of the test item using a qualified analytical procedure.

Results are presented in table 6 and figures 9 and 10

**Table 6: Summary of Tacrolimus Pharmacokinetic Parameters in Female Göttingen Minipig Whole Blood Following Subcutaneous Administration of Tacrolimus on Days 1, 29, 57 and 85**

| **Parameters** | **Dose Level** | | | | |
|---|---|---|---|---|---|
| | **F87** | | | **F72** | |
| | **10 mg/animal** | **20 mg/animal** | **40 mg/animal** | **20 mg/animal** | **40 mg/animal** |
| **Day 1** | | | | | |
| tₗₐₛₜ (h) | 672 | 672 | 670 | 672 | 671 |
| tₘₐₓ(h) | 72(72-96)^{$} | 96(48-168)^{$} | 48(24-168)^{$} | 48(10-72)^{$} | 48(24-240)^{$} |
| Cₘₐₓ (ng/mL) | 17.8 | 18.8 | 38.6 | 19.5 | 38.4 |
| Cₘₐₓ/D1 (ng/mL/mg/kg) | 44.9 | 22.7 | 24.9 | 24.5 | 24.5 |
| Cₘₐₓ/D2 (ng/mL/mg) | 1.78 | 0.939 | 0.964 | 0.976 | 0.961 |
| Cₗₐₛₜ (ng/mL) | 2.12 | 4.83 | 5.44 | 6.10 | 13.3 |
| AUCₗₐₛₜ (h.ng/mL) | 4760 | 6120 | 11100 | 6560 | 13000 |
| AUCₗₐₛₜ/D1 (h.ng/mL/mg/kg) | 12000 | 7390 | 7210 | 8190 | 8340 |
| AUCₗₐₛₜ/D2 (h.ng/mL/mg) | 476 | 306 | 277 | 328 | 326 |
| t_{1/2} (h) | 202 | 206' | 194 | n/a | 249' |
| Cₐᵥ(ng/mL) | 7.09 | 9.10 | 16.5 | 9.76 | 23.9 |
| Δ of concentrations (%) | 88.0 | 75.5 | 85.6 | 68.5 | 59.6 |

| **Day 29** | | | | | |
|---|---|---|---|---|---|
| tₗₐₛₜ(h) | 672 | 672 | 672(671-672)^{$} | 672 | 672 |
| tₘₐₓ(h) | 48 | 72(24-96)^{$} | 48(48-94.9)^{$} | 24(24-48)^{$} | 24(24-72)^{$} |
| Cₘₐₓ (ng/mL) | 24.2 | 23.3 | 44.3 | 32.7 | 54.1 |
| Cₘₐₓ/D1 (ng/mL/mg/kg) | 62.2 | 30.6 | 29.8 | 42.0 | 35.4 |
| Cₘₐₓ/D2 (ng/mL/mg) | 2.42 | 1.17 | 1.11 | 1.63 | 1.35 |
| Cₗₐₛₜ (ng/mL) | 3.49 | 4.86 | 8.95 | 7.48 | 16.0 |
| AUCₗₐₛₜ (h.ng/mL) | 5550 | 7820 | 13300 | 9810 | 15400 |
| AUCₗₐₛₜ/D1 (h.ng/mL/mg/kg) | 14200 | 10200 | 8880 | 12600 | 10100 |
| AUCₗₐₛₜ/D2 (h.ng/mL/mg) | 555 | 391 | 332 | 491 | 385 |
| t_{1/2}(h) | 222 | 226 | 225 | 235' | n/a |
| Cₐᵥ(ng/mL) | 8.26 | 11.6 | 19.8 | 14.6 | 22.9 |
| Δ of concentrations (%) | 85.1 | 79.3 | 79.1 | 76.9 | 69.9 |

| **Day 56** | | | | | |
|---|---|---|---|---|---|
| tₗₐₛₜ (h) | 671 | 671 | 671 | 671 | 671 |
| tₘₐₓ (h) | 72(24-96)^{$} | 48(24-48)^{$} | 72 | 24(4-24)^{$} | 24(24-48)^{$} |
| Cₘₐₓ(ng/mL) | 26.6 | 36.9 | 56.7 | 29.9 | 52.0 |
| Cₘₐₓ/D1 (ng/mL/mg/kg) | 69.4 | 49.5 | 37.4 | 39.4 | 35.0 |
| Cₘₐₓ/D2 (ng/mL/mg) | 2.66 | 1.85 | 1.42 | 1.50 | 1.30 |
| Cₗₐₛₜ(ng/mL) | 5.74 | 7.25 | 16.0 | 11.3 | 24.3 |
| AUCₗₐₛₜ (h.ng/mL) | 8030 | 12300 | 20600 | 10400 | 19300 |
| AUCₗₐₛₜ/D1 (h.ng/mL/mg/kg) | 21000 | 16500 | 13700 | 13700 | 13000 |
| AUCₗₐₛₜ/D2 (h.ng/mL/mg) | 803 | 614 | 516 | 520 | 481 |
| t_{1/2}(h) | n/a | 238 | 231 | n/a | n/a |
| Cₐᵥ (ng/mL) | 12.0 | 18.3 | 30.7 | 15.5 | 28.7 |
| Δ of concentrations (%) | 76.8 | 80.2 | 71.6 | 60.6 | 52.9 |

| **Day 85** | | | | | |
|---|---|---|---|---|---|
| tₗₐₛₜ (h) | 2830 | 2830 | 2830 | 2830 | 2830 |
| tₘₐₓ (h) | 48(24-96)^{$} | 48 | 48(24-168)^{$} | 24(10-48)^{$} | 24(24-240)^{$} |
| Cₘₐₓ(ng/mL) | 21.4 | 34.0 | 49.4 | 26.6 | 58.4 |
| Cₘₐₓ/D1 (ng/mL/mg/kg) | 58.6 | 48.3 | 34.1 | 35.8 | 40.4 |
| Cₘₐₓ/D2 (ng/mL/mg) | 2.14 | 1.70 | 1.24 | 1.33 | 1.46 |
| C₆₇₂(ng/mL) | 4.82 | 8.14 | 17.1 | 10.3 | 22.5 |
| Cₗₐₛₜ(ng/mL) | 1.95 | 5.61 | 12.6 | 3.27 | 11.7 |
| AUCₗₐₛₜ(h.ng/mL) | 13300 | 24100 | 45700 | 22000 | 55700 |
| AUCₗₐₛₜ/D1 (h.ng/mL/mg/kg) | 36500 | 34200 | 31600 | 29600 | 38000 |
| AUCₗₐₛₜ/D2 (h.ng/mL/mg) | 1330 | 1210 | 1140 | 1100 | 1390 |
| AUC₀₋₆₇₂ (h.ng/mL) | 7260 | 10900 | 17900 | 9900 | 22000 |
| AUC_{0.672}/D1 (h.ng/mL/mg/kg) | 19900 | 15500 | 12400 | 13400 | 15100 |
| AUC₀₋₆₇₂/D2 (h.ng/mL/mg) | 726 | 543 | 447 | 495 | 550 |
| t_{1/2} (h) | 668 | 574' | 801' | 1050 | n/a |
| Cₐᵥ(ng/mL) | 10.8 | 16.2 | 26.6 | 14.7 | 32.7 |
| Δ of concentrations (%)² | 76.9 | 76.6 | 65.5 | 60.5 | 55.7 |
| Total AUCₗₐₛₜ (h.ng/mL) | 31600 | 50400 | 90800 | 48700 | 103000 |
| Total AUCₗₐₛₜ/D1 (h.ng/mL/mg/kg) | 20700 | 16600 | 15100 | 15800 | 17200 |
| Total AUCₗₐₛₜ/D2 (h.ng/mL/mg) | 790 | 629 | 567 | 609 | 646 |
| Ratio Cₘₐₓ/D1 Day 29/Day 1 | 1.4 ± 0.3 | 1.4 ± 0.4 | 1.2 ± 0.4 | 1.8 ± 0.3 | 1.6 ± 0.3 |
| Ratio Cₘₐₓ/D1 Day 57/Day 29 | 1.2 ± 0.5 | 1.8 ± 0.8 | 1.3 ± 0.2 | 0.9 ± 0.2 | 1.0 ± 6.1 |
| Ratio Cₘₐₓ/D1 Day 85/Day 57 | 0.9 ± 0.3 | 1.0 ± 0.1 | 0.9 ± 0.2 | 0.9 ± 0.2 | 1.2 ± 0.7 |
| Ratio AUCₗₐₛₜ/D1 Day 29/Day 1 | 1.2 ± 0.1 | 1.4 ± 0.2 | 1.3 ± 0.3 | 1.6 ± 0.3 | 1.4 ± 0.6 |
| Ratio AUCₗₐₛₜ/D1 Day 57/Day 29 | 1.5 ± 0.5 | 1.8 ± 0.7 | 1.5 ± 0.2 | 1 ± 0.2 | 1.3 ± 0.1 |
| Ratio AUCₗₐₛₜ/D1 Dav 85³/Day 57 | 1.0 ± 0.5 | 0.9 ± 0.1 | 0.9 ± 0.1 | 1.0 ± 0.2 | 1.2 ± 0.4 |

| | | | | | |
|---|---|---|---|---|---|
| ^{$}: median (min-max); /D1: divided by the actual dose per kg; /D2: divided by the actual dose per animal; ¹: based on a single value; ²: Calculated with C₆₇₂. ³: AUC₍₀₋₆₇₂₎/D1 used instead of AUCₗₐₛₜ/D1. | | | | | |

Following each monthly subcutaneous injection of Tacrolimus at 10, 20 and 40 mg/animal of F87 or 20 and 40 mg/animal of F72 for 4 months, the blood concentrations of Tacrolimus were quantifiable throughout the 672-hour sampling period. In the 3-month follow up, quantifiable blood concentrations were observed up to Day 203.

The inter-variability in the blood concentrations, evaluated by %CV, was similar for both formulations with the average %CV ranging approx. from 13% to 60%.

Dose effect was evaluated by comparing the exposure parameters, Cₘₐₓ/D1 and AUCₗₐₛₜ/D1 or AUC₀₋₆₇₂/D1, at doses of 10 to 40 mg/animal of F87 or 20 to 40 mg/animal of F72. The area under the concentration vs. time curve (AUC) was calculated using the linear trapezoidal method. Values were compared to the preceding dose. Globally, the exposure increased in a less than dose-proportional manner for F87 and increased in a dose-proportional manner for F72.

As observed on figure 9 the profile shape of F87 showed a large peak followed by a slow decrease in blood levels whereas the profile shape of F72 showed a thinner peak followed by a faster decrease in blood levels then a more sustained release profile of tacrolimus. This was also demonstrated by a lower Δ of concentrations for F72 compared to F87. Globally, the exposure at 20 mg/animal or 40 mg/animal was similar between F72 and F87.

At steady state, whole blood levels of tacrolimus in the target range of 5-20 ng/mL and daily AUC in the target range of 130-475 ng.h/mL (*i.e.* daily AUC ratio of 475/130 = 3.65), corresponding to clinical efficacy in human (Advagraf, Product Information), were used to evaluate the *in vivo* performance of candidates in minipigs. Results are presented in figure 10.

### PK3: A 4-week Pharmacokinetic Study of Different Sustain-Release Formulations of Tacrolimus Administrated by the Subcutaneous Route in Female Göttingen Minipig with an 8-Week Follow-Up Period.

*In vivo* detailed set-up procedure:
Three Tacrolimus formulations were tested in a pharmacokinetic study in female Göttingen minipig of 9-14 months old and with a weight between 20 and 26 kg. Drug products containing 20 mg of Tacrolimus were subcutaneously administered in a skin fold caudal to the axillar region/shoulder of the minipig using 1 mL Soft Ject^{®} syringes and 21G needles. Injected formulations volumes were fixed at 90 or 175 µL depending on the initial Tacrolimus content in the formulations. One initial administration was made followed by a 2-month follow-up period. Blood samples were collected into EDTA tubes at predose and at different time points:
- After injection: T2h, T4h, T6h, T10h, T24h (Day 1), T48h (Day 2), T72h (Day 3), T96h (Day 4), T168h (Day 7), T240h (Day 10), T336h (Day 14), T408h (Day 17), T504h (Day 21), T576h (Day 24), T672h (Day 28). Whole blood samples were analyzed for concentration of test item using a qualified analytical procedure.
- During the 2-month follow-up period: Once a week until the end of the study (4 time points/ month), on Days 36, 43, 50, 57, 64, 71, 78 and 85.
Whole blood samples were analyzed to determine the concentration of the test item using a qualified analytical procedure.

Results are presented in table 7 and figures 11 and 12.

**Table 7: PK3**

| **Parameters** | **F70** | **F109** | **F117** |
|---|---|---|---|
| **Actual Dose Level (mg/animal)** | 19.6 ± 0.7 | 20.4 ± 0.5 | 22.4 ± 1.6 |
| **Actual Dose Level (mg/kg)** | 0.80 ± 0.03 | 0.84 ± 0.02 | 0.93 ± 0.05 |
| **Tₘₐₓ(h)** | 24 (24-48)^{$} | 48 (24-168)^{$} | 48 (24-240)^{$} |
| **Cmax (ng/mL)** | 30.0 ± 8.3 | 25.6 ± 11.9 | 16.8 ± 5.1 |
| **Cₘₐₓ/D1 (kg.ng/mL/mg)** | 37.9 ± 11.5 | 30.2 ± 13.6 | 17.9 ± 4.8 |
| **AUCₗₐₛₜ3M (h.ng/mL)** | 9006 ± 1494 | 10763 ± 2095 | 9286 ± 815 |
| **AUCₗₐₛₜ3M/D1 (h.kg.ng/mL/mg)** | 11320 ± 2022 | 12718 ± 2240 | 9929 ± 487 |
| **AUCₗₐₛₜ4M (h.ng/mL)** | - | - | 11329 ± 1370 |
| **AUC_{∞}(h.ng/mL)** | 8839 ± 2111 | 12737 ± 1232 | *Not calculated* |
| **t_{½}(h)** | 519 ± 388 | 690 ± 138 | *Not calculated* |

| | | | |
|---|---|---|---|
| ^{$}: median (range); /D1: divided by the actual dose per kg; - : not applicable. | | | |

Following subcutaneous injection of F70; F109 and F117, the blood concentrations of Tacrolimus were quantifiable up to the last sampling (Day 84 for F70 and F109 and Day 112 for F117). Similar AUCₗₐₛₜ 3M were obtained for all formulations but the lowest Cₘₐₓ and inter-variability in Tacrolimus level were obtained with F117.

The tacrolimus level remained within the 5 to 20 ng/mL narrow clinical target range for 28 days after injection only with F117, as shown on figure 11. Individual and mean daily AUC ratios were close to or below the threshold of 3.65 as represented on figure 12.

The present invention will be described in further detail with reference to the following clauses:
1. A pharmaceutical composition comprising
   (a) a triblock copolymer having the formula:

      PLAᵥ-PEG_{w}-PLAₓ

      wherein v and x are the number of repeat units ranging from 1 to 3,000 and w is the number of repeat units ranging from 3 to 300 and v=x or v≠x in an amount of 22 to 34 w/w % of the total composition;
   (b) a diblock copolymer having the formula:

      mPEG_{y}-PLA_{z}

      wherein y and z are the number of repeat units with y ranging from 2 to 250 and z ranging from 1 to 3,000 in an amount of 5 to 9 w/w% of the total composition;
   (c) tacrolimus or a pharmaceutically acceptable salt thereof in an amount of 8 to 32 w/w % of the total composition; and
   (d) organic solvent in an amount of 30 to 62 w/w % of the total composition.
2. A pharmaceutical composition for use in the therapeutic suppression of the immune system of a subject, wherein the use comprises administering the pharmaceutical composition to the subject, said composition comprising:
   (a) a triblock copolymer having the formula:

      PLAᵥ-PEG_{w}-PLAₓ

      wherein v and x are the number of repeat units ranging from 1 to 3,000 and w is the number of repeat units ranging from 3 to 300 and v=x or v≠x in an amount of 22 to 34 w/w % of the total composition;
   (b) a diblock copolymer having the formula:

      mPEG_{y}-PLA_{z}

      wherein y and z are the number of repeat units with y ranging from 2 to 250 and z ranging from 1 to 3,000 in an amount of 5 to 9 w/w % of the total composition;
   (c) tacrolimus or a pharmaceutically acceptable salt thereof in an amount of 10 to 30 w/w % of the total composition; and
   (d) organic solvent in an amount of 30 to 62 w/w % of the total composition.
3. A composition or composition for use according to clause 1 or clause 2 wherein for the triblock copolymer w is an integer from 20 to 25 and v and x are each an integer from 40 to 50, preferably wherein w is about 22 and v and x are each about 45; or wherein w is an integer from 20 to 25 and v and x are each an integer from 60 to 75, preferably wherein w is about 22 and v and x are each about 68.
4. A composition or composition for use according to according to any preceding clause wherein for the triblock copolymer the molecular weight of the PEG repeat unit is 1 kDa, and the lactic acid/ethylene oxide molar ratio is 4 or 6.
5. A composition or composition for use according to according to any preceding clause wherein for the diblock copolymer y is an integer from 7 to 10 and z is an integer from 60 to 85, preferably wherein y is about 8 and z is about 68; or wherein y is an integer from 43 to 47 and z is an integer from 126 to 141, preferably wherein y is about 45 and z is about 136.
6. A composition or composition for use according any preceding clause wherein for the diblock copolymer the molecular weight of the PEG repeat unit is 0.35 kDa and the lactic acid/ethylene oxide molar ratio is 8.5; or the molecular weight of the PEG repeat unit is 2kDa and the lactic acid/ethylene oxide molar ratio is 3.
7. A composition according to any of clauses 2 to 6 for the use according to any of clauses 2 to 6, wherein a plurality of doses of the composition are administered to the subject, optionally wherein the doses are administered over the lifetime of the subject.
8. A composition according to clause 7 for the use according to clause 7, wherein the time period between administration of consecutive doses is at least 14 days, preferably at least 21 days, most preferably at least 28 days.
9. A composition according to any of clauses 2 to 8 for the use according to any of clauses 2 to 8 wherein the composition is administered to the subject on a 4 weekly or monthly basis.
10. A composition according to any of clauses 2 to 9 for the use according to any of clauses 2 to 9 wherein the use comprises parenteral administration of the composition, preferably subcutaneous injection of the composition.
11. A composition according to any of clauses 2 to 10 for the use according to any of clauses 2 to 10 wherein the use comprises injecting the composition using a needle and syringe, optionally using an injection device.
12. A composition according to any of clauses 2 to 11 for the use according to any of clauses 2 to 11 wherein the dose of tacrolimus administered to the subject is controlled by adjusting the volume of the composition administered to the subject.
13. A composition according to any of clauses 2 to 12 for the use according to any of clauses 2 to 12 wherein the composition releases a therapeutic concentration of tacrolimus into the blood plasma of the subject for at least 14 days, preferably at least 21 days, most preferably at least 28 days after administration of the composition.
14. A composition according to clause 13 for the use according to clause 13 wherein the therapeutic concentration of tacrolimus in the blood plasma of the subject is 5 to 20 ng/mL, with the proviso that the concentration of tacrolimus can be higher than 20 ng/mL for a period of up to 48 hours.
15. A composition according to any of clauses 2 to 14 for the use according to any of clauses 2 to 14 wherein the use comprises maintaining the daily AUC in the range of from about 130 to 475 ng.h/mL
16. A composition according to any of clauses 2 to 15 for the use according to any of clauses 2 to 15 wherein the use comprises maintaining the daily AUC ratio below a maximum value in the range of from about 3.60 to about 3.70, preferably below about 3.65.
17. A composition according to any of clauses 2 to 16 for the use according to any of clauses 2 to 16 wherein the composition comprises a dose concentration of tacrolimus of from about 100 to about 350 mg/mL, preferably about 230 mg/mL or preferably about 115 mg/mL.
18. A composition according to any of clauses 2 to 17 for the use according to any of clauses 2 to 17 wherein the dose is administered in a volume of from about 0.1 mL to about 2 mL, optionally from about 0.1 mL to about 1.5 mL, optionally from about 0.1 mL to about 1 mL, optionally from about 0.1 mL to about 0.5 mL.
19. A composition according to any of clauses 2 to 18 for the use according to any of clauses 2 to 18, wherein the composition is injected over a period of about 1 second to about 2 minutes, optionally about 1 second to 1 minute, optionally about 1 second to about 30 seconds, optionally about 1 second to about 20 seconds, optionally about 1 second to about 10 seconds, optionally about 1 to about 5 seconds.
20. A composition according to any of clauses 2 to 19 for the use according to any of clauses 2 to 19, wherein the use comprises prophylaxis and/or treatment of allograft rejection in a subject who has received an allograft.
21. A composition according to clause 20 for the use according to clause 20 wherein the allograft is an organ, tissue or cells.
22. A composition according to clause 20 or clause 21 for the use according to clause 20 or clause 21 wherein the allograft is a liver, kidney or heart, preferably a kidney.
23. A composition according to any of clauses 2 to 19 for the use according to any of clauses 2 to 19 wherein the use comprises prophylaxis and/or treatment of an autoimmune disease, optionally wherein the use comprises prophylaxis and/or treatment of Lupus Nephritis.
24. A composition or composition for use according to any preceding clause which is suitable for forming a depot when injected into the body.
25. A composition or composition for use according to any preceding clause wherein the organic solvent is dimethyl sulfoxide.
26. A composition or composition for use according to any preceding clause wherein the weight ratio of the triblock copolymer to the diblock copolymer is about 4:1.
27. A composition or composition for use according to any preceding clause comprising about 10 to 30 w/w % tacrolimus, optionally about 10 to 20 w/w % tacrolimus, optionally about 20 w/w % tacrolimus.
28. A composition or composition for use according to any preceding clause comprising about 24 to 32 w/w % of the triblock copolymer, optionally about 26 to 30 w/w % of the triblock copolymer, optionally about 27 to 29 w/w % of the triblock copolymer, optionally about 28 w/w% of the triblock copolymer.
29. A composition or composition for use according to any preceding clause comprising, about 6 to 8 w/w% of the diblock copolymer, optionally about 7 to 8 w/w% of the diblock copolymer, optionally about 7 w/w% of the diblock copolymer.
30. A composition or composition for use according to any preceding clause comprising about 30 to 60 w/w%, optionally about 40 to 50 w/w%, optionally about 45 to 50 w/w%, optionally about 45 w/w% dimethyl sulfoxide.
31. A composition or composition for use according to any preceding clause which has a dynamic viscosity of from about 200 mPa.s. to about 3000 mPa.s, optionally from about 1000 mPa.s to about 3000 mPa.s, optionally from about 1000 to about 2000 mPa.s, optionally from about 1500 to about 1700 mPa.s.
32. A composition or composition for use according to any preceding clause, further comprising one or more pharmaceutically-acceptable excipients.
33. A composition or composition for use according to any preceding clause wherein for the triblock copolymer the molecular weight of the PEG repeat unit is 1 kDa and the lactic acid/ethylene oxide molar ratio is 4.
34. A composition or composition for use according to any of clauses 1 to 32 wherein for the triblock copolymer the molecular weight of the PEG repeat unit is 1 kDa and the lactic acid/ethylene oxide molar ratio is 6.
35. A composition or composition for use according to any preceding clause wherein for the diblock copolymer the molecular weight of the PEG repeat unit is 0.35 kDa and the lactic acid/ethylene oxide molar ratio is 8.5.
36. A composition or composition for use according to any of clauses 1 to 34 wherein for the diblock copolymer the molecular weight of the PEG repeat unit is 2 kDa and the lactic acid/ethylene oxide molar ratio is 3.
37. A composition or composition for use according to any of clauses 1 to 33 comprising 27-29 w/w % of the triblock copolymer, 6-8 w/w % of the diblock copolymer, 44-46 w/w % dimethyl sulfoxide, and 19-21 w/w % tacrolimus, and wherein for the triblock copolymer the molecular weight of the PEG repeat unit is 1 kDa and the lactic acid/ethylene oxide molar ratio is 4, and for the diblock copolymer the molecular weight of the PEG repeat unit is 2 kDa and the lactic acid/ethylene oxide molar ratio is 3.
38. A composition or composition for use according to any of clauses 1 to 33 comprising 23-25 w/w % of the triblock copolymer, 5-7 w/w % of the diblock copolymer, 59-61 w/w % dimethyl sulfoxide, and 9-11 w/w % tacrolimus, and wherein for the triblock copolymer the molecular weight of the PEG repeat unit is 1 kDa and the lactic acid/ethylene oxide molar ratio is 4, and for the diblock copolymer the molecular weight of the PEG repeat unit is 2 kDa and the lactic acid/ethylene oxide molar ratio is 3.
39. A method of therapeutic suppression of the immune system of a subject as defined in any preceding clause, the method comprising the step of administering a composition as defined in any preceding claim to the subject.
40. A method of preventing or treating allograft rejection in a subject who has received an allograft, the method comprising the step of administering a composition as defined in any preceding clause to the subject.

## Claims

1. A pharmaceutical composition comprising
(a) a triblock copolymer having the formula:
PLAᵥ-PEG_{w}-PLAₓ
wherein v and x are the number of repeat units ranging from 1 to 3,000 and w is the number of repeat units ranging from 3 to 300 and v=x or v≠x in an amount of 22 to 34 w/w % of the total composition;
(b) a diblock copolymer having the formula:
mPEG_{y}-PLA_{z}
wherein y and z are the number of repeat units with y ranging from 2 to 250 and z ranging from 1 to 3,000 in an amount of 5 to 9 w/w% of the total composition;
(c) tacrolimus or a pharmaceutically acceptable salt thereof in an amount of 8 to 32 w/w % of the total composition; and
(d) organic solvent in an amount of 30 to 62 w/w % of the total composition.

2. A pharmaceutical composition for use in the therapeutic suppression of the immune system of a subject, wherein the use comprises administering the pharmaceutical composition to the subject, said composition comprising:
(a) a triblock copolymer having the formula:
PLAᵥ-PEG_{w}-PLAₓ
wherein v and x are the number of repeat units ranging from 1 to 3,000 and w is the number of repeat units ranging from 3 to 300 and v=x or v≠x in an amount of 22 to 34 w/w % of the total composition;
(b) a diblock copolymer having the formula:
mPEG_{y}-PLA_{z}
wherein y and z are the number of repeat units with y ranging from 2 to 250 and z ranging from 1 to 3,000 in an amount of 5 to 9 w/w % of the total composition;
(c) tacrolimus or a pharmaceutically acceptable salt thereof in an amount of 8 to 32 w/w % of the total composition; and
(d) organic solvent in an amount of 30 to 62 w/w % of the total composition.

3. A composition or composition for use according to claim 1 or claim 2 wherein for the triblock copolymer w is an integer from 20 to 25 and v and x are each an integer from 40 to 50, preferably wherein w is about 22 and v and x are each about 45; or wherein w is an integer from 20 to 25 and v and x are each an integer from 60 to 75, preferably wherein w is about 22 and v and x are each about 68; and/or wherein for the triblock copolymer the molecular weight of the PEG repeat unit is 1 kDa, and the lactic acid/ethylene oxide molar ratio is 4 or 6.

4. A composition or composition for use according to according to any preceding claim wherein for the diblock copolymer y is an integer from 7 to 10 and z is an integer from 60 to 85, preferably wherein y is about 8 and z is about 68; or wherein y is an integer from 43 to 47 and z is an integer from 129 to 141, preferably wherein y is about 45 and z is about 136; and/or wherein for the diblock copolymer the molecular weight of the PEG repeat unit is 0.35 kDa and the lactic acid/ethylene oxide molar ratio is 8.5; or the molecular weight of the PEG repeat unit is 2kDa and the lactic acid/ethylene oxide molar ratio is 3.

5. A composition according to any of claims 2 to 4 for the use according to any of claims 2 to 4, wherein a plurality of doses of the composition are administered to the subject, optionally wherein the doses are administered over the lifetime of the subject, optionally wherein the time period between administration of consecutive doses is at least 14 days, preferably at least 21 days, most preferably at least 28 days, or wherein the composition is administered to the subject on a 4 weekly or monthly basis.

6. A composition according to any of claims 2 to 5 for the use according to any of claims 2 to 5 wherein the use comprises parenteral administration of the composition, preferably subcutaneous injection of the composition; and/or wherein the use comprises injecting the composition using a needle and syringe, optionally using an injection device; and/or wherein the dose of tacrolimus administered to the subject is controlled by adjusting the volume of the composition administered to the subject.

7. A composition according to any of claims 2 to 6 for the use according to any of claims 2 to 6 wherein the composition releases a therapeutic concentration of tacrolimus into the blood plasma of the subject for at least 14 days, preferably at least 21 days, most preferably at least 28 days after administration of the composition, optionally wherein the therapeutic concentration of tacrolimus in the blood plasma of the subject is 5 to 20 ng/mL, with the proviso that the concentration of tacrolimus can be higher than 20 ng/mL for a period of up to 48 hours; and/or wherein the use comprises maintaining the daily AUC in the range of from about 130 to 475 ng.h/mL; and/or wherein the use comprises maintaining the daily AUC ratio below a maximum value in the range of from about 3.60 to about 3.70, preferably below about 3.65.

8. A composition according to any of claims 2 to 7 for the use according to any of claims 2 to 7 wherein the composition comprises a dose concentration of tacrolimus of from about 100 to about 350 mg/mL, preferably about 230 mg/mL or preferably about 115 mg/mL; and/or wherein the dose is administered in a volume of from about 0.1 mL to about 2 mL, optionally from about 0.1 mL to about 1.5 mL, optionally from about 0.1 mL to about 1 mL, optionally from about 0.1 mL to about 0.5 mL; and/or wherein the composition is injected over a period of about 1 second to about 2 minutes, optionally about 1 second to 1 minute, optionally about 1 second to about 30 seconds, optionally about 1 second to about 20 seconds, optionally about 1 second to about 10 seconds, optionally about 1 to about 5 seconds.

9. A composition according to any of claims 2 to 8 for the use according to any of claims 2 to 8, wherein the use comprises prophylaxis and/or treatment of allograft rejection in a subject who has received an allograft.

10. A composition according to claim 9 for the use according to claim 9 wherein the allograft is an organ, tissue or cells, optionally wherein the allograft is a liver, kidney or heart, preferably a kidney.

11. A composition according to any of claims 2 to 8 for the use according to any of claims 2 to 8 wherein the use comprises prophylaxis and/or treatment of an autoimmune disease, optionally wherein the use comprises prophylaxis and/or treatment of Lupus Nephritis.

12. A composition or composition for use according to any preceding claim which is suitable for forming a depot when injected into the body.

13. A composition or composition for use according to any preceding claim wherein the organic solvent is dimethyl sulfoxide; and/or wherein the weight ratio of the triblock copolymer to the diblock copolymer is about 4:1; and/or comprising about 10 to 30 w/w % tacrolimus, optionally about 10 to 20 w/w % tacrolimus, optionally about 20 w/w % tacrolimus; and/or comprising about 24 to 32 w/w % of the triblock copolymer, optionally about 26 to 30 w/w %, optionally about 27 to 29 w/w % of the triblock copolymer, optionally about 28 w/w% of the triblock copolymer; and/or comprising about 6 to 8 w/w% of the diblock copolymer, optionally comprising about 7 to 8 w/w% of the diblock copolymer, optionally about 7 w/w% of the diblock copolymer; and/or comprising about 30 to 60 w/w%, optionally about 40 to 50 w/w%, optionally about 45 to 50 w/w%, optionally about 45 w/w% dimethyl sulfoxide; and/or which has a dynamic viscosity of from about 200 mPa.s. to about 3000 mPa.s, optionally from about 1000 mPa.s to about 3000 mPa.s, optionally from about 1000 to about 2000 mPa.s, optionally from about 1500 to about 1700 mPa.s; and/or further comprising one or more pharmaceutically-acceptable excipients.

14. A composition or composition for use according to any preceding claim wherein for the triblock copolymer the molecular weight of the PEG repeat unit is 1 kDa and the lactic acid/ethylene oxide molar ratio is 4.

15. A composition or composition for use according to any of claims 1 to 13 wherein for the triblock copolymer the molecular weight of the PEG repeat unit is 1 kDa and the lactic acid/ethylene oxide molar ratio is 6.

16. A composition or composition for use according to any preceding claim wherein for the diblock copolymer the molecular weight of the PEG repeat unit is 0.35 kDa and the lactic acid/ethylene oxide molar ratio is 8.5.

17. A composition or composition for use according to any of claims 1 to 15 wherein for the diblock copolymer the molecular weight of the PEG repeat unit is 2 kDa and the lactic acid/ethylene oxide molar ratio is 3.

18. A composition or composition for use according to any of claims 1 to 13 comprising 27-29 w/w % of the triblock copolymer, 6-8 w/w % of the diblock copolymer, 44-46 w/w % dimethyl sulfoxide, and 19-21 w/w % tacrolimus, and wherein for the triblock copolymer the molecular weight of the PEG repeat unit is 1 kDa and the lactic acid/ethylene oxide molar ratio is 4, and for the diblock copolymer the molecular weight of the PEG repeat unit is 2 kDa and the lactic acid/ethylene oxide molar ratio is 3.

19. A composition or composition for use according to any of claims 1 to 13 comprising 23-25 w/w % of the triblock copolymer, 5-7 w/w % of the diblock copolymer, 59-61 w/w % dimethyl sulfoxide, and 9-11 w/w % tacrolimus, and wherein for the triblock copolymer the molecular weight of the PEG repeat unit is 1 kDa and the lactic acid/ethylene oxide molar ratio is 4, and for the diblock copolymer the molecular weight of the PEG repeat unit is 2 kDa and the lactic acid/ethylene oxide molar ratio is 3.

20. A method of therapeutic suppression of the immune system of a subject as defined in any preceding claim, the method comprising the step of administering a composition as defined in any preceding claim to the subject.

21. A method of preventing or treating allograft rejection in a subject who has received an allograft, the method comprising the step of administering a composition as defined in any preceding claim to the subject.
